# EUROPÄISCHE PATENTSCHRIFT

(11) **EP 2 977 009 B1**
(45) Veröffentlichungstag und Bekanntmachung des Hinweises auf die Patenterteilung: **13.05.2020**
(21) Anmeldenummer: 15176352.1
(22) Anmeldetag: 09.05.2012
(51) Int. Cl.: A61B 6/00

(54) **DURCHSTRAHLUNGSSYSTEM UND KALIBRIERUNG DESSELBEN**
RADIATION SYSTEM AND CALIBRATION OF THE SAME
SYSTEME DE RAYONNEMENT ET SON ETALONNAGE

(30) Priorität: 09.05.2011 DE 102011075527
(43) Veröffentlichungstag der Anmeldung: 27.01.2016
(62) Teilanmeldung aus: 12723409.4
(73) Patentinhaber: Fraunhofer-Gesellschaft zur Förderung der angewandten Forschung e.V., 80686 München (DE)
(72) Erfinder: Jobst, Andreas, 90491 Nürnberg (DE); Kostka, Günther, 91056 Erlangen (DE); Schmitt, Peter, 91058 Erlangen (DE)
(74) Vertreter: Schenk, Markus

(56) Entgegenhaltungen:
- EP-A1- 1 078 599
- WO-A1-2004/066612
- WO-A2-2008/054717
- US-A1- 2007 041 508

## Beschreibung

Die vorliegende Erfindung bezieht sich auf ein Durchstrahlungssystem, wie z.B. Computertomographen oder dergleichen, und eine Kalibrierung solcher Systeme.

Durchstrahlungssysteme arbeiten häufig mit Röntgenstrahlung. Flächendetektoren von Durchstrahlungssystemen besitzen beispielsweise einen Schirm, wie z.B. einen Szintillatorschirm für röntgenempfindliche Flächendetektoren, welcher dann mit einer oder mehreren optischen Kameras aufgenommen wird. Auf diese Weise existieren beispielsweise Röntgenkameras, die in der digitalen Radioskopie oder Computertomographie beispielsweise für die Qualitätskontrolle von Produkten oder für die Metrologie eingesetzt werden.

Bedingt durch deren Aufbau besitzen konventionelle bildgebende Röntgenflächenkameras, wie z.B. Flachbilddetektoren auf Basis von amorphem Silizium, in der Regel quadratische Pixel, die die aktive Detektorfläche mehr oder weniger lückenlos ausfüllen, d.h. die Szintillatorfläche lückenlos abtasten. Die Ausrichtung der Zeilen und Spalten sowie ihre Abstände sind bei diesen Röntgenkameras bauartbedingt fest vorgegeben und können nicht geändert werden. Ausnahme ist das sogenannte Binning, d.h. das Zusammenfassen mehrerer benachbarter Pixel zu einem Bildpunkt, bei dem die Auflösung und die Datenmenge reduziert und das Signal-Rausch-Verhältnis verbessert werden kann.

Für bestimme Anwendungen dieser Röntgenkameras ist es notwendig, die Kamera mechanisch exakt so in einer Anlage zu justieren, dass die Zeilen und Spalten eine definierte Orientierung relativ zu anderen Komponenten der Anlage, beispielsweise der mechanischen Verfahrachsen besitzen. Mit sinkender Pixelgröße bzw. steigender Auflösung der Kamera steigen auch die Anforderungen an die Genauigkeit und Stabilität der mechanischen Justierung zwischen der Kamera und den anderen Anlagen-Komponenten, wie z.B. dem Manipulator, der beispielsweise ein Roboterarm, ein C-Gestell oder dergleichen umfasst.

Die Justage von Durchstrahlungssystemen war deshalb bisher immer recht aufwendig und umfasste einen iterativen und approximativen Prozess, in welchem abwechselnd Durchstrahlungen an Kalibrierobjekten durchgeführt und die so erhaltenen Messungen zur Rejustage des Flächendetektors verwendet wurden.

Dokument US 2007/0041508 zeigt eine Kalibrierung eines Durchstrahlungssystems mittels eines Kalibrierungsmusters und erwähnt eine automatische Korrektur der aufgenommenen Bilder.

Die Aufgabe der vorliegenden Erfindung besteht darin, ein Durchstrahlungssystem sowie ein Verfahren und eine Vorrichtung zum Kalibrieren desselben zu schaffen, so dass ein besserer Kompromiss zwischen Justageaufwand auf der einen und Messgenauigkeit auf der anderen Seite erhalten werden kann, wie z.B. gleiche Messgenauigkeit bei geringerem Justageaufwand, und dies zudem mit geringem zusätzlichen Aufwand an Hard- und/oder Software.

Diese Aufgabe wird durch den Gegenstand der unabhängigen Ansprüche des beigefügten Anspruchssatzes gelöst.

Eine Erkenntnis der vorliegenden Erfindung besteht darin, erkannt zu haben, dass es möglich ist, Lagefehler des Flächendetektors in dem Achsensystem des Durchstrahlungssystems relativ zu einer Solllage, die nach beispielsweise einem Grobaufbau des Durchstrahlungssystems verbleiben, auszugleichen und damit die Genauigkeit des Durchstrahlungssystems fast beliebig genau an diejenige Genauigkeit heranzubringen, die resultiert hätte, wenn der Flächendetektor anschließend noch mechanisch exakt genau justiert worden wäre, wenn eine geometrische Korrektur des Durchstrahlungsrohbilds durchgeführt wird, so dass eine Veränderung bzw. ein Fehler, wie. z.B. eine Verzeichnung, eine Lageabweichung und Größenabweichung oder dergleichen, einer Projektion des durchstrahlten Objekts in dem Durchstrahlungsrohbild aufgrund des Lagefehlers verringert bzw. korrigiert wird.

Gemäß Ausführungsbeispielen wird die Erkenntnis ausgenutzt, dass Durchstrahlungssysteme häufig bereits Flächendetektoren verwenden, die in der Lage sind, Durchstrahlungsrohbilder zu erzeugen, die eine höhere Auflösung besitzen als die Durchstrahlungsbilder, die nach Vorverarbeitung tatsächlich verwertet werden sollen. Dadurch ist es möglich, Lagefehler des Flächendetektors in dem Achsensystem des Durchstrahlungssystems relativ zu einer Solllage, die nach beispielsweise einem Grobaufbau des Durchstrahlungssystems verbleiben, noch besser auszugleichen bzw. die Genauigkeit des Durchstrahlungssystems fast beliebig genau an diejenige Genauigkeit heranzubringen, die resultiert hätte, wenn der Flächendetektor anschließend noch mechanisch exakt genau justiert worden wäre, und zwar indem eine geometrische Korrektur des Durchstrahlungsrohbilds, so dass eine Veränderung einer Projektion des durchstrahlten Objekts in dem Durchstrahlungsrohbild aufgrund des Lagefehlers verringert bzw. korrigiert wird, in einer Auflösung durchgeführt wird, die höher als eine Auflösung des schließlich benötigten Durchstrahlungsbilds ist.

Gemäß einem Ausführungsbeispiel kann das Durchstrahlungssystem einen Rekonstruierer aufweisen, der ausgebildet ist, das Objekt aus Durchstrahlungsbildern zu rekonstruieren, die aus einer Mehrzahl von Durchstrahlungsrohbildern durch Vorverarbeitung gewonnen worden sind. In diesem Fall bestehen unterschiedliche Möglichkeiten für die Solllage des Flächendetektors relativ zu dem Achsensystem des Durchstrahlungssystems. Das Achsensystem kann definiert sein durch die Strahlachse zwischen Strahlungsquelle und Flächendetektor, eine Rotationsachse des Manipulators, eine Translationsverfahrachse des Manipulators, eine Trajektorie des Flächendetektors zwischen der Erzeugung der Durchstrahlungsrohbilder, die später für eine Rekonstruktion verwendet werden, und zwar durch all diese Achsen, einen Teil derselben oder lediglich eine dieser Achsen, und die Solllage kann vorsehen, dass der Flächendetektor zu diesen Achsen senkrecht stehen soll, oder dass eine Spalten- oder Zeilenrichtung des Flächendetektors parallel zu einer dieser Achsen ausgerichtet sein soll bzw. dass die Durchstrahlungsbilder, die beispielsweise eine regelmäßige Pixelarrayanordnung aus Zeilen und Spalten aufweisen, mit ihren Zeilen- und/oder Spaltenrichtungen mit einer jeweiligen der Achsen des Achsensystems parallel ausgerichtet sind, oder dass ein vorbestimmter Winkel eingehalten wird.

Gemäß einem Ausführungsbeispiel weist der Flächendetektor eine Mehrzahl von Flächendetektorarrays zur Erzeugung von Teilbildern auf, die unter gegenseitigem Überlapp zusammen jeweils ein Durchstrahlungsrohbild ergeben. In diesem Fall kann die geometrische Korrektur zusammen mit dem Zusammenfügen bzw. Stitching der Teilbilder und/oder unter gleichzeitiger Entzerrung der Teilbilder durchgeführt werden. Bei einer Kalibrierung des Durchstrahlungssystems können beispielsweise die Korrekturdaten, die der Vorverarbeitung zugrunde liegen, zunächst vorkalibriert sein, um das Stitching und ggf. die teilbildindividuelle Entzerrung auszuführen, während eine Modifikation der Kalibrierdaten nochmals nach dem Aufbau des Durchstrahlungssystems durchgeführt wird, um eventuelle Lagefehler des Flächendetektors auszugleichen und somit den mechanischen Kalibrieraufwand bzw. Aufstellungsaufwand oder Zusammenbauaufwand des Durchstrahlungssystems zu verringern.

Gemäß einem weiteren Ausführungsbeispiel wird von obigen Erkenntnissen Gebrauch gemacht, indem einem Durchstrahlungssystem mit äquiangularer Pixelanordnung eine einfache Art und Weise zur Verstellbarkeit des Detektor/Quell-Abstands verliehen wird, indem obige geometrische Korrektur abhängig von dem eingestellten Abstand durchgeführt wird, um ein äquiangulares Durchstrahlungsbild zu erhalten. Die Korrekturdaten können auf eine ähnliche Weise bestimmt werden, so dass dieselben ggf. gleichzeitig andere Korrekturen durchführen können als lediglich die einen Übergang von einer beispielsweisen Anordnung der Pixel des Flächendetektors in Spalten und Zeilen zu der äquiangularen Pixelanordnung, wie z.B. einen Verkippungsausgleich oder dergleichen.

Bevorzugte Ausführungsbeispiele der vorliegenden Erfindung werden nachfolgend Bezug nehmend auf die beiliegenden Zeichnungen erläutert. Es zeigen:
- Fig. 1: ein schematisches Blockschaltbild eines Durchstrahlungssystem gemäß einem Ausführungsbeispiel;
- Fig. 2: ein Flussdiagramm eines Verfahrens zur Kalibrierung eines Durchstrahlungssystems gemäß Fig. 1;
- Fig. 3: eine schematische Seitenschnittansicht eines Flächendetektors gemäß einem Ausführungsbeispiel;
- Fig. 4: einen schematischen Ausschnitt der Verteilung von Pixeln bzw. Pixeldetektorpositionen in einer Bildebene des Flächendetektors von Fig. 3 gemäß einem exemplarischen Ausführungsbeispiel;
- Fig. 5: eine schematische Seitenschnittansicht zur Veranschaulichung eines Beispiels für einen Lagefehler des Flächendetektors;
- Fig. 6: links ein Durchstrahlungsbild bei verkipptem Flächendetektor ohne geometrische Korrektur und rechts das Durchstrahlungsbild bei korrekt angeordnetem Flächendetektor;
- Fig. 7: eine schematische Darstellung zur Veranschaulichung einer möglichen Konfiguration zur Erzeugung von Durchstrahlungsrohbildern zur Kalibrierung eines Durchstrahlungssystems gemäß einem Ausführungsbeispiel;
- Fig. 8: simulierte Sequenzen von Durchstrahlungsrohbildern mit einer Kugel als Kalibrierobjekt in einem Durchstrahlungssystem gemäß Fig. 7, und zwar links bei verkipptem Detektor, in der Mitte bei ideal justiertem Detektor und rechts die Differenz der beiden Bilder;
- Fig. 9: simulierte Durchstrahlungsrohbilder eines Liniengitters zur Verdeutlichung des Auflösungsverlustes durch geometrische Transformation, die in der Auflösung der Durchstrahlungsbilder durchgeführt wird;
- Fig. 10: Grauwertprofile über das Liniengitter von Fig. 9;
- Fig. 11: eine schematische Darstellung eines Durchstrahlungssystems zur Veranschaulichung eines Vergleichs zwischen einer äquidistanten und einer äquiangularen Pixelanordnung; und
- Fig. 12: Simulationen von Durchstrahlungsbildern, einmal mit einem Detektor mit quadratischen Pixeln, und das anderen Mal mit variierender Pixelbreite.

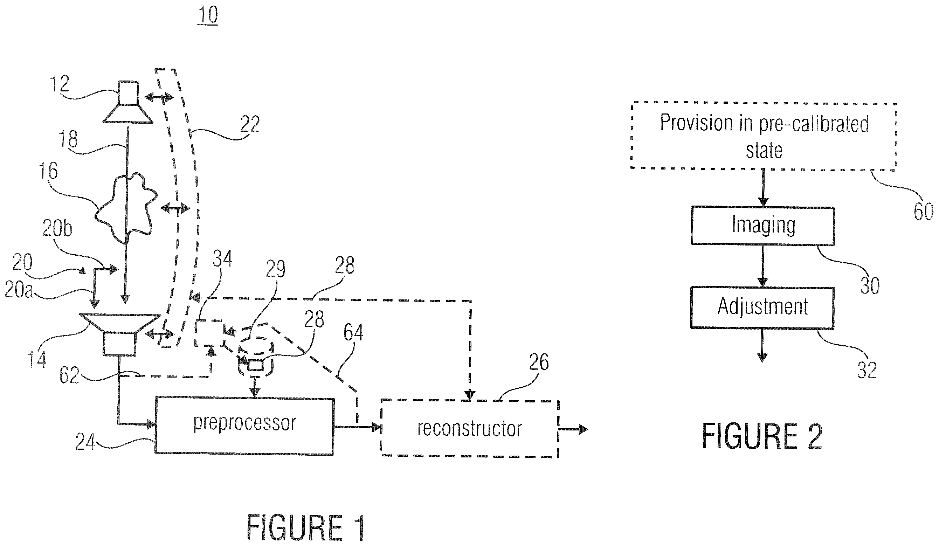

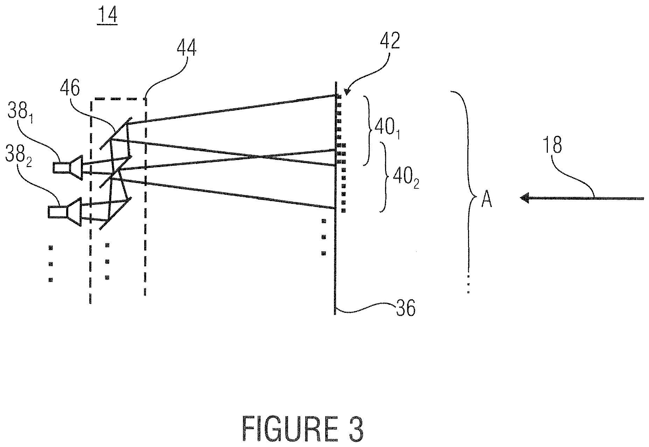

Figur 1 zeigt ein Durchstrahlungssystem 10 gemäß einem Ausführungsbeispiel der vorliegenden Anmeldung. Das Durchstrahlungssystem von Figur 1 umfasst eine Strahlungsquelle 12, wie z.B. eine Röntgenquelle, und einen Flächendetektor 14 zum Erzeugen eines Durchstrahlungsrohbildes eines Objektes 16 auf Basis von Strahlung 18, wie z.B. Röntgenstrahlung, die das Objekt 16 zwischen der Strahlungsquelle 12 und dem Flächendetektor 14 durchdringt. Wie später Bezug nehmend auf Figur 3 beschrieben werden wird, kann der Flächendetektor 14 beispielsweise einen Szintillatorschirm aufweisen, der die Strahlung 18 ortsaufgelöst in optische Information umwandelt, welcher Schirm wiederum von einer oder mehreren optischen Kameras ggf. über einen oder mehrere Spiegel zum Schutz der Kameras vor der Strahlung 18 aufgenommen wird. Dem Flächendetektor 14 ist eine Bildebene zuordenbar und weist in dieser Bildebene eine Verteilung von Pixeln bzw. Pixelpositionen auf. Der Szintillatorschirm des Flächendetektors 14 bildet beispielsweise diese Bildebene, wobei die Pixelpositionen in der Bildebenen durch die Verteilung der Pixel der optischen Kameras in deren durch das Kameraobjektiv definierte Objektebene, d.h. dem Szintillatorschirm, definiert sind.

Das Durchstrahlungssystem 10 ist so konstruiert, dass durch dasselbe ein Achssystem festgelegt ist. Das Achssystem ist in Figur 1 exemplarisch mit 20 angedeutet. Wie es aus der nachfolgenden Beschreibung deutlich werden wird, kann das Achssystem beispielsweise eine Strahlachse 20a aufweisen, entlang der die beispielsweise parallelisierte Strahlung 18 auf den Flächendetektor 14 trifft, oder eine Bewegungsachse 20b eines optionalen Manipulators 22 des Durchstrahlungssystems 10, der ausgebildet sein kann, eine relative Lage zwischen der Strahlungsquelle 12, dem Objekt 16 und dem Flächendetektor 14 zu ändern.

Das Durchstrahlungssystem 10 umfasst ferner einen Vorverarbeiter 24 zum Vorverarbeiten des Durchstrahlungsrohbilds zu einem Durchstrahlungsbild, das eine Auflösung besitzt, die geringer als eine Auflösung des Durchstrahlungsrohbilds ist, wie es von dem Flächendetektor 14 erzeugt worden ist. Insbesondere führt der Vorverarbeiter 14 die Vorverarbeitung des Durchstrahlungsrohbilds zu dem Durchstrahlungsbild unter geometrischer Korrektur des Durchstrahlungsrohbilds in einer Auflösung durch, die höher als eine Auflösung des Durchstrahlungsbilds ist, so dass eine Veränderung einer Projektion des Objekts in dem Durchstrahlungsrohbild aufgrund eines Lagefehlers des Flächendetektors 14 in dem Achsensystem 20 relativ zu einer Solllage verringert wird. Das bedeutet in etwa Folgendes: der Flächendetektor 14 weist beispielsweise eine strahlungsempfindliche Fläche A in der Bildebene auf, und zwar abgetastet in N Pixeln in der Bildebene. Der Quotient N/A kann als Maß für die Auflösung der durch den Flächendetektor 14 erzeugten Durchstrahlungsrohbilder angesehen werden. Das Durchstrahlungssystem 10 verwertet aber nachher Durchstrahlungsbilder, die eine niedrigere Auflösung besitzen, d.h. eine Auflösung, bei der die strahlungssensitive Fläche A des Flächendetektors 14 mit weniger Pixeln n < N abgetastet wird. Wie es im Folgenden noch erörtert werden wird, kann es sich bei der Verteilung der Pixel sowohl der Durchstrahlungsrohbilder als auch der Durchstrahlungsbilder um eine regelmäßige Verteilung der Pixel in Spalten und Zeilen handeln, wobei vorzugsweise die Verteilung in den Durchstrahlungsbildern regelmäßig, wie z.B. kartesisch oder affin, ist, wohingegen die Verteilung in den Durchstrahlungsrohbildern eine Unregelmäßigkeit aufweisen kann, wie es später Bezug nehmend auf Figur 3 exemplarisch beschrieben wird.

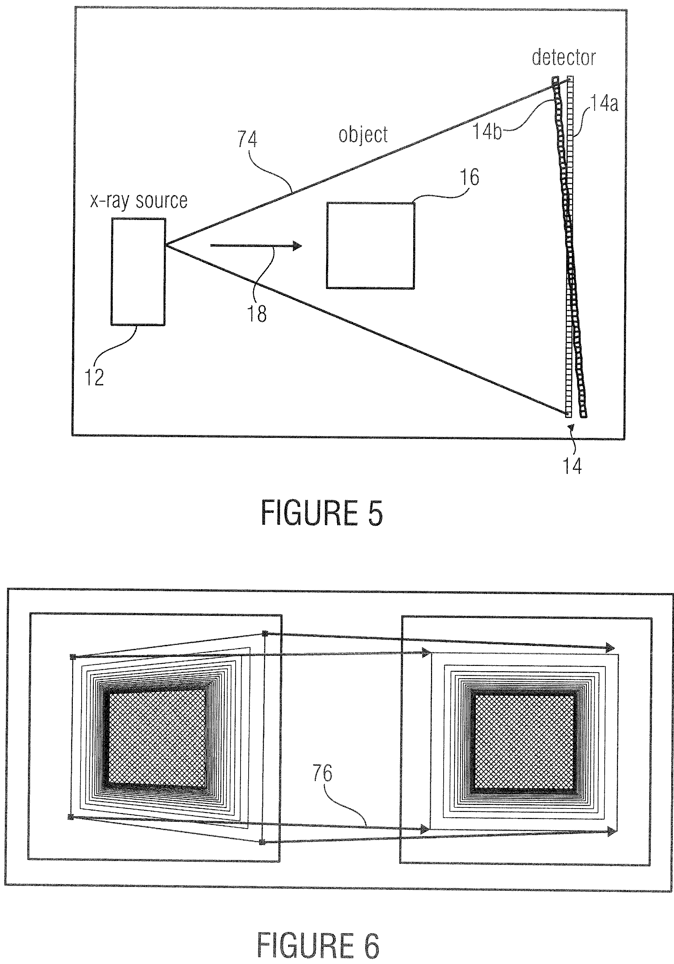

Das Durchstrahlungssystem 10 ist aber dazu vorgesehen, Durchstrahlungsbilder zu erzeugen, die eine Projektion des Objekts 16 auf eine Bildebene des Flächendetektors 14 in einem Zustand darstellen, bei dem sich dieselbe in einer gewissen Solllage befindet, wie z.B. senkrecht zu der vorerwähnten Strahlachse 20a oder anderen beispielhaften Solllagen relativ zu dem Achssystem 20. Ist die Bildebene des Flächendetektors 14 gegenüber dieser Solllage verkippt, so erscheint die Projektion des Objekts 16 auf die Bildebene verzerrt, wofür ein Beispiel in Figur 6 gezeigt ist, und ist die Bildebene an einer falschen Position entlang einer der Achsen, wie z.B. zu weit entfernt von der Strahlungsquelle 12 angeordnet, oder ist sie verdreht gegenüber einer der Achsen, so kann die Projektion des Objekts 16 in den Durchstrahlungsrohbildern beispielsweise zentrisch gestreckt bzw. gestaucht oder gedreht erscheinen, was Nachteile bei der nachfolgenden Verwertung der Durchstrahlungsbilder haben kann, wie z.B. einem Rekonstruierer 26 des Durchstrahlungssystems 10, der beispielsweise ausgebildet ist, um aus einer Mehrzahl von Durchstrahlungsbildern, die durch den Vorverarbeiter 24 aus Durchstrahlungsrohbildern erhalten worden sind, die von dem Flächendetektor 14 unter Änderung der relativen Lage zwischen Strahlungsquelle 12, Objekt 16 und Flächendetektor 14 zwischen den Durchstrahlungsrohbildern erzeugt worden sind, das Objekt zu rekonstruieren und dabei von der Solllage des Detektors 14 ausgeht. Der Rekonstruierer 26 rekonstruiert beispielsweise auf Basis der Durchstrahlungsbilder und von Informationen über die relative Lage zwischen Strahlungsquelle 12, Objekt 16 und Flächendetektor 14, in welcher diese durch Strahlungsbilder bzw. die denselben zugrunde liegenden Durchstrahlungsrohbilder erzeugt worden sind, eine räumliche Verteilung von beispielsweise der Dichte des Objekts, eine Verteilung des Auftretens eines bestimmten Materials oder eine Verteilung der Dichte bzw. des Auftretens einer bestimmten Atomordnungszahl in dem Objekt, also allgemeiner ausgedrückt, eine räumliche Materialeigenschaftsverteilung des Objekts.

Das Durchstrahlungssystem 10 von Figur 1 kompensiert nun eine Fehljustage bzw. den Lagefehler des Flächendetektors 14 relativ zu der vorbeschriebenen Solllage dadurch, dass die geometrische Korrektur des Durchstrahlungsrohbilds von dem Flächendetektor 14 in einer Auflösung stattfindet, die höher als die Auflösung des jeweiligen Durchstrahlungsbilds ist, das aus der Vorverarbeitung in dem Vorverarbeiter 24 resultiert. In anderen Worten wird die vorerwähnte Verzeichnung, zentrische Streckung oder Drehung des Objekts in der Bildebene transformativ in einer Auflösung bei der Vorverarbeitung berücksichtigt, die höher ist als die schließliche Endauflösung der Durchstrahlungsbilder, die schließlich verwertet werden, wie z.B. von dem optionalen Rekonstruierer 26.

Bevor Bezug nehmend auf Fig. 2 ein Verfahren zur Kalibrierung des Durchstrahlungssystems 10 von Figur 1 beschrieben wird, wird darauf hingewiesen, dass es sich bei dem optionalen Manipulator 22 beispielsweise um ein C-Gestell handeln kann, an welchem Strahlungsquelle 12 und Flächendetektor 14 einander über eine Rotationsachse gegenüberliegend montiert sind, um die wiederum das C-Gestell drehbar gelagert sein kann, um das beispielsweise feststehende Objekt 16 aus unterschiedlichen Richtungen durchleuchten zu können. Bei dem Manipulator 22 kann es sich beispielsweise aber auch um einen Roboterarm handeln oder um zwei Roboterarme, einen für die Strahlungsquelle 12 und einen anderen für den Flächendetektor 14, oder der Manipulator 22 weist zusätzlich oder alternativ einen Translationsverfahrweg oder dergleichen auf. Die Strahlung 18, die von der Strahlungsquelle 12 ausgegeben wird, kann parallelisiert sein oder divergent aufgefächert sein. Wie es durch einen gestrichelten Pfeil 28 dargestellt ist, kann es sein, dass der optionale Rekonstruierer 26 mit dem Manipulator 22 gekoppelt ist, um für jedes Durchstrahlungsbild Kenntnis darüber zu haben, in welcher relativen Lagebeziehung sich Strahlungsquelle 12, Objekt 16 und Flächendetektor 14 befanden, als das jeweilige Durchstrahlungsrohbild zu dem jeweiligen Durchstrahlungsbild erzeugt worden ist. Der Rekonstruierer 26 rekonstruiert dann aus den mehreren Durchstrahlungsbildern zu unterschiedlichen relativen Lagen das Objekt 16, d.h. rekonstruiert seine räumliche Dichteverteilung oder dergleichen. Es existieren allerdings auch andere Möglichkeiten für eine Ausgestaltung des Durchstrahlungssystems 10, wofür im Folgenden noch Beispiele geliefert werden.

Schließlich soll noch darauf hingewiesen werden, dass der Vorverarbeiter 24 in festverdrahteter Form, in programmierbarer Logik oder in Software implementiert sein kann, wobei Gleiches für den Rekonstruierer 26 gilt. Ein Computer kann beispielsweise die Aufgaben des Vorverarbeiters 24 und des Rekonstruierers 26 übernehmen. Der Vorverarbeiter 24 kann allerdings auch beispielsweise in einem Gehäuse des Flächendetektors 14 oder zwischen dem Rekonstruierer 26 und dem Flächendetektor 14 positioniert sein.

Figur 2 zeigt nun eine Vorgehensweise zur Kalibrierung des Durchstrahlungssystems 10 von Figur 1 gemäß einem Ausführungsbeispiel. Dabei wird davon ausgegangen, dass der Vorverarbeiter 24 die Vorverarbeitung dadurch durchführt, dass er auf bestimmte Korrekturdaten 28 zurückgreift bzw. diese Korrekturdaten 28 auf die Durchstrahlungsrohbilder des Flächendetektors 14 anwendet. Wie es in Figur 1 angedeutet ist, können die Korrekturdaten 28 beispielsweise in einem Speicher 29 lokalisiert sein, auf den der Vorverarbeiter 24 zugreift, um die Korrekturdaten 28 zu erhalten, abhängig von welchen dann der Vorverarbeiter 24 die Vorverarbeitung durchführt. Der Speicher 29 kann beispielsweise ein nicht flüchtiger Speicher sein, wie z.B. ein einmal beschreibbarer Nur-Lese-Speicher. Es soll aber darauf hingewiesen werden, dass die Korrekturdaten 28 auch in einer eventuellen Softwareimplementierung des Vorverarbeiters 24, d.h. im Programmcode selbst, integriert sein könnten.

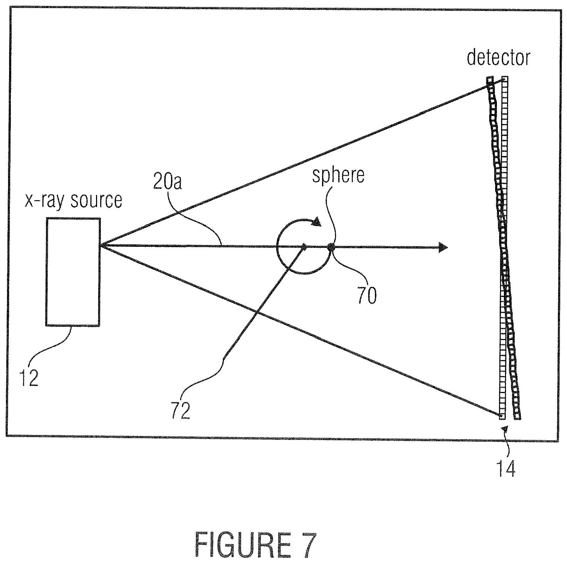

Das Verfahren von Figur 2 beginnt mit der Erzeugung zumindest eines Durchstrahlungsrohbildes eines Kalibrierobjektes 16 durch den Flächendetektor 14. Wie es später noch Bezug nehmend auf Figur 7 beschrieben wird, kann es sich beispielsweise bei dem Kalibrierobjekt um eine Kugel aus für die Strahlung 18 stark absorbierenden Material handeln, und es kann sein, dass mehrere Durchstrahlungsrohbilder dieses Kalibrierobjektes aufgenommen werden, wobei zwischen denselben der Manipulator 22 die Relativlage zwischen Strahlungsquelle 12, Kalibrierobjekt und Flächendetektor 14 verändert, wie z.B. bei fester einander gegenüberliegender Lage von Strahlungsquelle 12 zu Flächendetektor 14. Der Manipulator 22 dreht letztgenanntes Paar beispielsweise um eine Rotationsachse, die von der Kugel lediglich so weit entfernt ist, dass in jeder Drehposition des Strahlungsquellen/Flächendetektor-Paars zur Rotationsachse das Kalibrierobjekt vollständig auf die flächensensitive Fläche des Flächendetektors 14 projiziert wird bzw. in den Durchstrahlungsrohbildern enthalten ist.

Nach diesem in Figur 2 als Bilderzeugung bezeichneten Schritt 30 folgt ein Schritt 32 der Einstellung der Korrekturdaten 28 des Vorverarbeiters 24 abhängig von dem zumindest einen Durchstrahlungsrohbild des Schritts 30, so dass die Anwendung der eingestellten Korrekturdaten 28 zu einer geometrischen Korrektur von Durchstrahlungsrohbildern des Flächendetektors 14 führt, und zwar in einer Auflösung, die eben höher als die Auflösung der Durchstrahlungsbilder ist, und so, dass die vorerwähnte Veränderung der Projektion von Objekten in den Durchstrahlungsrohbildern erhalten wird. Dabei kann der Schritt des Einstellens 32 beispielsweise das erstmalige Speichern der Korrekturdaten 28 in den Speieher 29 oder das Überschreiben bereits vorhandener (vorkalibrierter) Korrekturdaten mit den neuen Korrekturdaten umfassen. Der Schritt des Einstellens 32 kann beispielsweise das Berechnen der Korrekturdaten 28 über eine parametrisierte Rechenvorschrift aufweisen, oder das Nachschlagen in einer Tabelle. Der Schritt 32 kann ferner das Extrahieren von ausgezeichneten Punkten, wie z.B. einem Mittelpunkt des Kalibrierobjekts, in dem oder den Durchstrahlungsrohbildern aus dem Schritt 30 umfassen bzw. die Ermittelung von deren Position in dem- oder denselben, um aus diesen Positionen die neuen Korrekturdaten für die Einstellung im Schritt 32 zu ermitteln.

Das System 10 von Figur 1 kann eine Vorrichtung zur Durchführung des Verfahrens von Figur 2 selbst umfassen. Sie ist in Figur 1 mit 34 angedeutet. Sie ist mit dem Speicher 29 verbunden und erhält wie oben erwähnt zum Beispiel die Rohbilder am Ausgang des Detektors 14 direkt oder verwendet durch die voreingestellten Korrekturdaten 28 vorkalibrierte Bilder am Ausgang des Detektors 14 zur Einstellung in Schritt 32.

Nachdem im Vorhergehenden der prinzipielle Aufbau und die Funktionsweise des Durchstrahlungssystems von Figur 1 und dessen Kalibrierung beschrieben worden ist, soll im Folgenden anhand von detaillierten Ausführungsbeispielen für die einzelnen Komponenten auf mögliche Implementierungsdetails eingegangen werden, die das Verständnis verbessern können.

So kann es sich beispielsweise bei dem vorbeschriebenen Lagefehler des Flächendetektors 14 in dem Achssystem 20 relativ zu der Solllage beispielsweise um eine Verkippung des Flächendetektors 14 bzw. einer Bildebene derselben aus einer senkrechten Ausrichtung zu einer Strahlachse 20a zwischen Strahlungsquelle 12 und Flächendetektor 14 handeln. Die Strahlachse 20a ist in dem Fall einer Parallelisierung des Strahls 18 durch die Richtung des Strahls 18, wie er von der Strahlungsquelle 12 emittiert wird, definiert. In dem Fall eines divergenten Strahlungsbündels 18, der von der Strahlungsquelle 12 ausgegeben wird, kann die Strahlachse 20a aber auch entlang desjenigen Strahls 18 definiert sein, der senkrecht durch beispielsweise eine Rotationsachse des Manipulators 22 oder senkrecht zu einer Translationsebene des Manipulators 22 führt, um die bzw. entlang der der Manipulator 22 das Paar aus Strahlungsquelle 12 und Flächendetektor 14 bewegt, wie z.B. weil der Manipulator 22 mechanisch so konstruiert ist, dass diese Rotationsachse bzw. diese Translationsebene konstruktiv vorgegeben ist, oder durch eine Trajektorie vorgegeben ist, entlang welcher Strahlungsquelle 12 und Flächendetektor 14 sich während der Erzeugung von Durchstrahlungsrohbildern bewegen sollen, die dann für eine eventuelle Rekonstruktion durch den Rekonstruierer 26 verwendet werden.

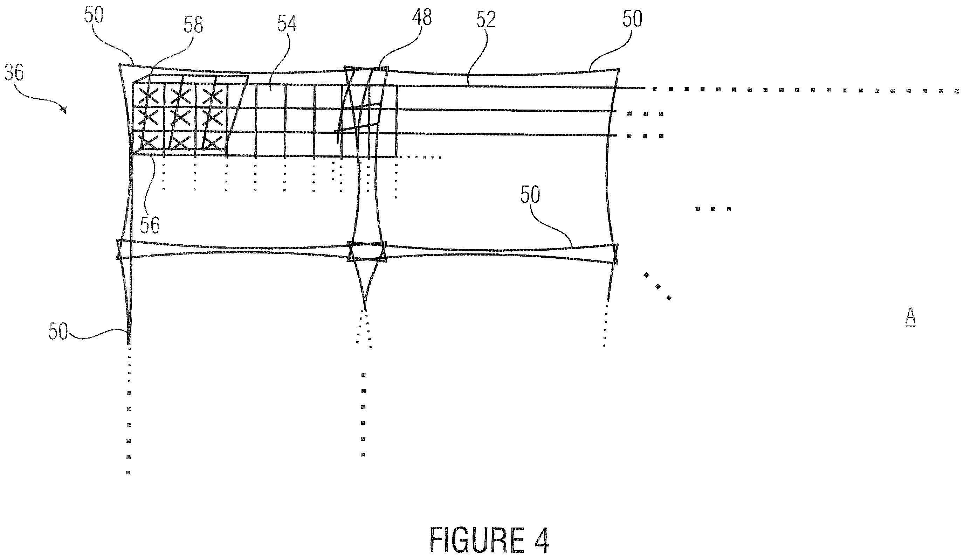

Zusätzlich oder alternativ kann es sich bei dem vorerwähnten Lagefehler des Flächendetektors 14 um eine Verkippung einer Spalten- oder Zeilenrichtung des Flächendetektors 14 relativ zu einer Rotations- oder Translationsverfahrachse des Manipulators 22 handeln, d.h. um eine Abweichung von einer parallelen Ausrichtung zur Selbigen. Weist der Flächendetektor 14 keine Unregelmäßigkeit in der Anordnung der Pixelpositionen in der Bildebene auf, so entspricht die erwähnte Spalten- und Zeilenrichtung den entlang dieser Richtungen angeordneten Pixelpositionen des Flächendetektors 14 in der Bildebene. In dem Fall des später Bezug nehmend auf Figur 3 beschriebenen Beispiels für eine Verteilung der Pixelpositionen in der Bildebene, entspricht die erwähnte Spalten- bzw. Zeilenrichtung beispielsweise einer gemittelten Spalten- bzw. Zeilenrichtung der tatsächlichen Pixelpositionen des Flächendetektors 14 in der Bildebene bzw. Projektionsebene oder aber einer Verteilung der Pixelpositionen des Flächendetektors 14 der Bildebene in einem Vorkalibrierungszustand, wie er im Folgenden noch Bezug nehmend auf Figuren 3 und 4 beschrieben wird. Bei der erwähnten Translationsverfahrachse kann es sich beispielsweise um die Translationsachse handeln, entlang der der Manipulator 22, Strahlungsquelle 12 und Flächendetektor 14 in einer Helixtrajektorie bewegt.

Bei dem vorerwähnten Lagefehler des Flächendetektors kann es sich aber auch um eine Verkippung in der soeben erwähnten Spalten- oder Zeilenrichtung des Flächendetektors 14 relativ zu einer Trajektorie handeln, die der Flächendetektor 14 relativ zu dem Objekt 16 zwischen der Mehrzahl von Durchstrahlungsrohbildern bzw. deren Erzeugung abfährt. Bei der Trajektorie kann es sich beispielsweise um die soeben erwähnte Helixbahn handeln.

Bevor nun im Folgenden auf weitere mögliche Details eingegangen wird, wie die einzelnen Komponenten des Durchstrahlungssystems 10 von Figur 1 aufgebaut sind und zusammenwirken, wie z.B. im Sinne eines Durchstrahlungsrekonstruktionssystems, wird im Folgenden Bezug nehmend auf Figuren 3 und 4 auf ein Beispiel dafür eingegangen, dass der Flächendetektor 14 möglicherweise eine Unregelmäßigkeit in der Verteilung der Pixelpositionen in der Bildebene aufweist. Figur 3 zeigt ein solches Beispiel für einen Flächendetektor 14. Der Flächendetektor 14 von Figur 3 umfasst einen Szintillatorschirm 36 und mehrere Kameras 38₁ und 38₂. Die Kameras 38₁ und 38₂ sind so eingesellt, dass ihre Schärfentiefenebene bzw. ihr Schärfentiefenbereich mit der Szintillatorebene des Szintillatorschirms 36 zusammenfällt, und so, dass die Bildausschnitte 40₁ und 40₂, die von den einzelnen Kameras 38₁ und 38₂ aufgenommen werden, die vorerwähnte strahlungsempfindliche Fläche A des Szintillatorschirms lückenlos abdecken. Dabei wird es bevorzugt, wie es in Figur 3 gezeigt ist, dass sich die Abschnitte 40₁ und 40₂ benachbarter Kameras 38₁ und 38₂ gegenseitig überlappen. Die Bilder der Pixel der Pixelarrays (nicht gezeigt) der Kameras 38₁ und 38₂, die über die Objektive (nicht gezeigt) dieser Kamera in der Objektebene derselben liegen, die vorzugsweise wiederum mit dem Szintillatorschirm 36 zusammenfällt, definieren also in dem Fall der Kamera von Figur 3 die vorerwähnte Verteilung 42 von Pixelpositionen in der Bildebene der Kamera 14.

Aufgrund des Überlapps und aufgrund von Positionierungsungenauigkeiten zwischen den Kameras 38₁ und 38₂ weist die Verteilung 42 Unregelmäßigkeiten auf gegenüber einer beispielsweise der Verteilung der Pixel in den Pixel-Arrays der Kameras 38₁ und 38₂ entsprechenden regelmäßigen Verteilung in Spalten und Zeilen.

Eingebaut wird die Kamera 14 in das Durchstrahlungssystem 10 so, dass die Strahlung 18 auf den Szintillatorschirm 36 trifft, wie zum Beispiel im Wesentlichen senkrecht, wie es im Vorhergehenden beschrieben worden ist, und dass dort die Strahlung in eine optische Informationsverteilung umgewandelt wird, wo dieselbe durch die Kameras 38₁ und 38₂ mit der Verteilung 42 der Pixel lokal abgetastet wird.

Wie es bei 44 exemplarisch gezeigt ist, kann die Flächenkamera 14 ein Spiegel-Array aus Spiegeln 46 umfassen, über welche die Kameras 38₁ und 38₂ den Szintillatorschirm 36 mittels Spiegelungen an den Spiegeln 46 auf ihr jeweiliges Pixel-Array abbilden, um so ggf. beispielsweise gegenüber der energiehöheren Strahlung 18 geschützt zu sein.

Bezug nehmend auf Figur 4 soll anhand des Beispiels von Fig. 3 der Zusammenhang zwischen der unregelmäßigen Verteilung 42 von Pixeln, den Durchstrahlungsrohbildern sowie den Durchstrahlungsbildern beschrieben werden. Figur 4 zeigt die Pixelverteilungen in der Bildebene bzw. der Projektionsebene der Flächenkamera 14, d. h. beispielsweise in der Ebene des Szintillatorschirms 36. Wie gesagt, sind in dem strahlungssensitiven Flächenteil A die Pixelpositionen 48 der Flächenkamera 14 verteilt, d. h. in dem Fall von Figur 3 die Bilder der Pixel der Pixel-Arrays der Kameras 38₁ und 38₂. Wie es mit kissenförmigen Rechtecken 50 angedeutet ist, deckt beispielsweise das Gesichtsfeld jeder einzelnen Kamera mit seinen Pixeln 48 einen Bereich 50 ab, der mit den Bereichen 50 benachbarter Kameras lateral überlappt, wie zum Beispiel über 0,1% bis 3% der Anzahl der Pixel der jeweiligen Kamera in der jeweiligen Richtung der Überlappung. Wie es in Fig. 4 angedeutet ist, können auch die Teilbereiche 50 regelmäßig in Spalten und Zeilen angeordnet sein.

Wie es bereits aber im Vorhergehenden erwähnt worden ist, weicht die Verteilung der Pixel 48 in der Bildebene der Kamera 14 von einer rein regelmäßigen Verteilung in Spalten und Zeilen aufgrund von Verzeichnungen durch die Objektive der Kameras 38₁ und 38₂, aufgrund von Verkippungen und Verdrehungen der Kameras und/oder aufgrund von gegenseitigen Positionsungenauigkeiten der Kameras 38₁ und 38₂ parallel zur Bildebene 36 ab. Zum leichteren Verständnis sind die Verzeichnungen in Fig. 4 ein wenig übertrieben dargestellt. Auch das Verhältnis der Größe der Pixel zu den Teilbereichen 50 wurde des einfachen Verständnis halber vergrößert dargestellt.

Werden nun Aufnahmen mit der Flächenkamera 14 von Fig. 3 und 4 durchgeführt, so können die Teilbilder 50 der Einzelkameras nicht einfach zusammengefügt werden, um ein sinnvolles Bild zu ergeben. Die Teilbilder müssen vielmehr zueinander ausgerichtet bzw. zusammengefügt werden, was auch als Stitching bezeichnet wird. Gleichzeitig können die Verzeichnungen korrigiert werden usw., bis sich durch entsprechende Interpolation aus den ursprünglichen Pixelwerten der Pixel 48 eine Abtastung des strahlungssensitiven Flächenanteils A in einem Pixelraster 52 ergeben, das beispielsweise regelmäßig verteilte Pixel 54 in Spalten und Zeilen aufweist, wie es in Fig. 4 angedeutet ist. Die Auflösung des Pixelrasters 52 kann beispielsweise gleich oder geringfügig kleiner als die Auflösung der Verteilung 42 der Pixel 48 sein und beispielsweise größer als die Endauflösung der Durchstrahlungsbilder.

Um die Abtastung in dem Pixelraster 52 zu erhalten, wird beispielsweise in Korrekturdaten für jedes Pixel 54 des Pixelrasters 52 definiert, wie benachbarte und beispielsweise mit dem jeweiligen Pixel 54 überlappende Pixel 48 gewichtet miteinander zu kombinieren bzw. addieren sind, um den jeweiligen Pixelwert des jeweiligen Pixels 54 zu ergeben.

Letztgenannter Übergang von der unregelmäßigen Verteilung 42 zu der regelmäßigen Verteilung 52 könnte exemplarisch bereits in Verarbeitungsrichtung vor dem Verarbeiter 24 beispielsweise bereits in der Flächenkamera 14 durchgeführt werden. Im Folgenden werden aber noch Ausführungsbeispiele beschrieben, bei denen diese Verarbeitung in dem Vorverarbeiter 24 selbst durchgeführt wird, wobei die Korrekturdaten dann modifiziert werden, um auch die geometrische Korrektur durchzuführen, wie sie sich ergibt, um die eventuellen Lagefehler des Flächendetektors 14 in dem Durchstrahlungssystem 10 auszugleichen. Wenn aber der Übergang zu der regelmäßigen Verteilung 52 bereits vor dem Vorverarbeiter 24 durchgeführt worden ist, dann handelt es sich beispielsweise bei dieser Verteilung 52 der Pixel 54 um die Durchstrahlungsrohbilder, die von der Flächenkamera in den Vorverarbeiter 24 eingehen. Die Auflösung der Durchstrahlungsrohbilder ist aber, wie es im Vorhergehenden erwähnt wurde, höher als die der Durchstrahlungsbilder, die von dem Vorverarbeiter 24 schließlich auszugeben sind, wie zum Beispiel an einen optionalen Rekonstruierer 26. Wenn der Flächendetektor 14 nun korrekt an seiner Solllage im Achsensystem 20 eingebaut wäre, dann müsste der Vorverarbeiter 24 beispielsweise lediglich ein Binning durchführen, d.h. ein Zusammenfassen benachbarter Pixel 54 des regelmäßigen Pixelrasters 52, um auf diese Weise eine Auflösungsreduktion durchzuführen, wobei dieses Binning in Figur 4 exemplarisch mit einem 3x3-Binning angedeutet ist, so dass jeweils 3x3-Blöcke von Pixeln 53 ein Pixel 56 eines Durchstrahlungsbildes am Ausgang des Vorverarbeiters 24 ergäben. Nun ist aber aufgrund des Lagefehlers des Flächendetektors 14 die Projektion des Objekts 16 in die Ebene 36 geometrisch verändert, wie zum Beispiel verzerrt, zentrisch gestreckt oder dergleichen, und die vorerwähnten Korrekturdaten 28 sind deshalb dazu vorgesehen, beim Übergang in die niedrigere Auflösung der Durchstrahlungsbilder die geometrische Veränderung wieder zu korrigieren, aber eben nicht in lediglich der Auflösung der Durchstrahlungsbilder mit den Pixeln 56, sondern in einer höheren Auflösung, wie zum Beispiel bereits in der Auflösung des Pixelrasters 52. Beispielsweise stellt die Vorrichtung 34 in dem Schritt 32 fest, dass die Pixel 54, die zusammen das größere Pixel 56 ergeben, so wie bei 58 angedeutet in der Bildebene 36 zu verschieben wären, um eine Abtastung der Durchstrahlung 18 zu ergeben, die derjenigen entspräche, wenn der Flächendetektor 14 in seiner Soll-Lage positioniert gewesen wäre. Die Korrekturdaten 28 können nun unterschiedliche Informationen beinhalten. Beispielsweise legen die Korrekturdaten 28 für jedes Pixel 56 des Durchstrahlungsbildes gemäß einer Vorschrift, die unter den Pixeln des Durchstrahlungsbildes gemäß der geometrischen Korrektur variiert, benachbarte Pixel 54 des Durchstrahlungsrohbildes fest, und wie dieselben zu dem jeweiligen Pixel 56 des Durchstrahlungsbildes zu kombinieren sind. Dabei ist es wichtig darauf hinzuweisen, dass die Gewichtung der Pixel 54 in der Auflösung des Pixelrasters 52, also in einer höheren Auflösung als der Auflösung der schließlichen Durchstrahlungsbilder, variiert. Die Korrekturdaten 28 könnten beispielsweise für jede verschobene Position der einzelnen Pixel 54 eine neue Interpolation durchführen, und dann das Binning. So ist es durch Unterteilung des Pixel 56 in Figur 4 angedeutet. Ein anderes Vorgehen ist aber ebenfalls möglich, wonach der flächige Überlapp der jeweiligen Pixel 54 mit dem zur geometrischen Korrektur verschobenen Pixel 58 ausgewertet wird, um den Gewichtungsbeitrag für das schließliche Durchstrahlungsbild zu erhalten.

Wie bereits erwähnt, wird es aber mehr bevorzugt, wenn der Vorverarbeiter 24 direkt die Durchstrahlungsrohbilder in Form der Pixelinformationen der Pixel 48 in der Verteilung 42 erhält. Der Vorverarbeiter 24 ist in diesem Fall für die kompletten Korrekturmaßnahmen zuständig, d. h. das vorerwähnte Stitching, die Verzeichnungskorrektur und Lagekorrektur für die Kameras 38₁ und 38₂ innerhalb des Flächendetektors 14 sowie die geometrische Korrektur zur virtuellen Lagekorrektur des Flächendetektors 14 in dem Achssystem 20 des Durchstrahlungssystems 10. Im Folgenden wird Bezug nehmend auf Figur 2 ein bevorzugtes Verfahren zur Kalibrierung des Durchstrahlungssystems 10 von Figur 1 für diesen Fall beschrieben, d. h. für den Fall eines eine Unregelmäßigkeit der Pixelverteilung in der Bildebene aufweisenden Flächendetektors 14, wobei der Vorverarbeiter 24 direkt die Durchstrahlungsrohbilder mit dieser Unregelmäßigkeit erhält.

Bei der nachfolgenden Beschreibung eines Kalibrierverfahrens wird also davon ausgegangen, dass der Flächendetektor 14 eine Unregelmäßigkeit in der Verteilung von Pixelpositionen in der Bildebene bzw. Projektionsebene des Flächendetektors 14 aufweist, wie sie beispielsweise aus den Umständen herrührt, wie sie Bezug nehmend auf Figur 3 und 4 erörtert worden sind. Das Verfahren weist in diesem Fall beispielsweise zusätzlich zu den Schritten 30 und 32 im Vorfeld derselben den Schritt der Bereitstellung 60 des Flächendetektors 14 in einem vorkalibrierten Zustand auf, bei dem die Korrekturdaten 28 so voreingestellt sind, dass dieselben die Unregelmäßigkeit der Verteilung der Pixelpositionen in der Bildebene korrigieren, so dass nämlich die Anwendung der so eingestellten Korrekturdaten 28 dazu führt, dass das Durchstrahlungsbild beim Ausgang des Vorverarbeiters 24 ein regelmäßiges Pixelraster aufweist, wie z.B. eben das Pixelraster der Pixel 56, wie es in Figur 4 angedeutet ist. Die Bilderzeugung in Schritt 30 wird in diesem vorkalibrierten Zustand durchgeführt, allerdings evtl. ohne Binning. Dies ermöglicht folgenden Vorteil: Die Vorrichtung 34 verwendet nicht die reinen Rohbilder, die sich aus den Teilbildern 50 zusammensetzen, wie es mit dem Pfeil 62 in Figur 1 angedeutet ist und wie es natürlich ebenfalls möglich wäre, sondern die Vorrichtung 34 verwendet zur Neueinstellung der Korrekturdaten 28 die in der einen oder den mehreren Bestrahlungen in dem Schritt 30 gewonnenen Durchstrahlungsrohbilder in einer durch die voreingestellten Korrekturdaten 28 im Hinblick auf die Unregelmäßigkeit der Verteilung der Pixelpositionen in der Bildebene korrigierten Form, wie es durch den Pfeil 64 in Figur 1 angedeutet ist. Das heißt, die Berechnung der Modifikation der Korrekturdaten 28 in dem Schritt 32 durch die Vorrichtung 34 vereinfacht sich, da die Berechnung davon ausgehen kann, dass die zugrunde gelegten Informationen bereits in einem regelmäßigen Pixelraster vorliegen, wie z.B. dem Pixelraster 52 aus Figur 4, oder sogar dem groben Pixelraster mit dem Pixel 56. Auf den Schritt 32 hin, liegen die Korrekturdaten 28 dann in einer modifizierten Form vor, die beispielsweise für jedes verschobene Grobpixel 58 der Durchstrahlungsbilder angeben, wie die zugrunde liegenden Urpixel 48 in der Bildebene 38 mit welcher Gewichtung zu kombinieren sind, um das jeweilige Pixel 56 zu ergeben. In anschließenden Durchstrahlungsmessungen erfolgt die Anwendung der Korrekturdaten 28 dann nur einmalig, und zwar zum Zwecke der geometrischen Korrektur der Lageabweichung des Flächendetektors 14 in dem Achsensystem 20, dem Stitching der Teilbilder 50 und der Verzeichnungskorrektur dieser Teilbilder, wie es im Vorhergehenden beschrieben worden ist.

Bezugnehmend auf vorhergehende Beschreibung wird noch darauf hingewiesen, dass es zwar bevorzugt ist, wenn die Auflösung der Durchstrahlungsrohbilder höher ist als diejenige der schließlich zu verwendenden Durchstrahlungsbilder, dass dies aber nicht notwenig ist. Gemäß weniger bevorzugten Ausführungsbeispielen ist dies nicht der Fall und die Korrektur findet einfach in der Auflösung all dieser Bilder statt, d.h. in der Auflösung der Durchstrahlungsrohbilder und Durchstrahlungsbilder.

In Bezug auf die vorhergehende Beschreibung wird auch darauf hingewiesen, dass das Durchstrahlungssystem gegebenenfalls zusätzlich so ausgestaltet sein könnte, dass beispielsweise der Quell/Detektor-Abstand verstellbar ist, wie z.B. zur Erzielung unterschiedlicher Vergrößerungen. Wenn dann noch das Pixelraster der schließlich zu verwendenden Durchstrahlungsbilder äquiangular ist, dann sind zusätzliche Korrekturen nach jeder Umstellung des Abstands erforderlich, wie z.B. zwischen zwei Messungen, wenn der Abstand umgestellt wird. Die Korrekturdaten können dann wie in Fig. 2 gezeigt ermittelt werden, wie z.B. vorab für unterschiedliche Abstände, oder jedes Mal nach einer Verstellung des Abstands neu. Echte Lagefehler des Detektors würden gegebenenfalls, d.h. optional, gleich mit ausgeglichen werden. Somit beschreibt Fig. 1 auch ein Durchstrahlungssystem mit einer Strahlungsquelle 12 zum Erzeugen von Strahlung in divergenter Weise; einem Flächendetektor 14 zum Erzeugen eines Durchstrahlungsrohbildes eines Objekts 16 auf Basis der Strahlung 18, die das Objekt 16 zwischen der Strahlungsquelle 12 und dem Flächendetektor 14 durchdringt, wobei das Durchstrahlungssystem so konstruiert ist, dass durch dasselbe ein Achssystem 20 festgelegt ist, und so, dass ein Abstand zwischen Strahlungsquelle und Flächendetektor verstellbar ist; einem Vorverarbeiter 24 zum Verarbeiten des Durchstrahlungsrohbilds zu einem äquiangularen Durchstrahlungsbild, und zwar abhängig von dem eingestellten Abstand. Umstellungen des zu Rede stehenden Abstands wären dann ohne weiteres möglich.

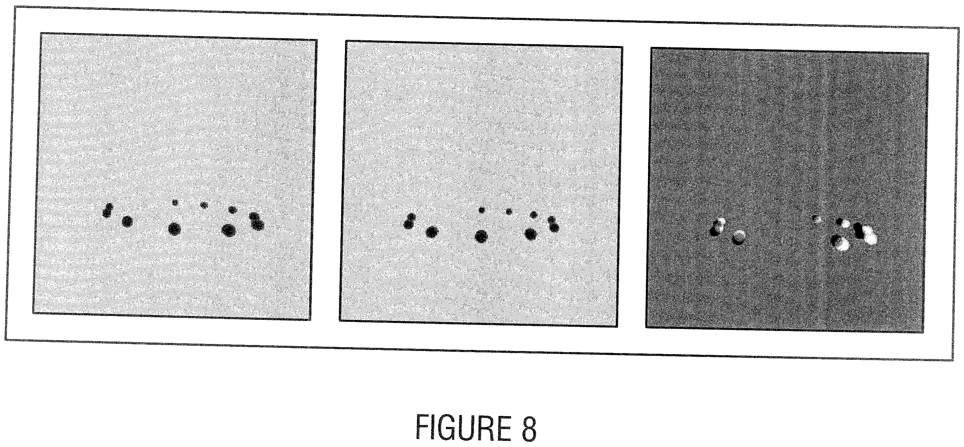

In der vorhergehenden Beschreibung von Fig. 2 wurde bezüglich des Schrittes 32 nicht speziell auf Möglichkeiten eingegangen, wie die Korrekturdaten 28 bestimmt werden können, in die ja indirekt oder direkt eine Bestimmung des Lagefehlers einfließt. Im Folgenden wird beispielsweise noch Bezug nehmend auf die Figuren 7 und 8 auf eine Möglichkeit eingegangen, einen Lagefehler des Flächendetektors 14 zu bestimmen, der in Flächennormalen verkippt zu einem Zustand ist, bei dem der Flächendetektor 14 in seiner Solllage wäre. Als Maß für die Verkippung werden dann beispielsweise zwei Kippwinkel erhalten, die dann in geeignete geometrische Verzeichungsparameter als die Korrekturdaten umgerechnet werden können. Wie es in Bezug auf Fig. 7 und 8 beschrieben werden wird, kann in diesem Fall als Kalibrierobjekt eine Kugel aus absorbierendem Material verwendet werden, die exzentrisch zu einer Rotationsachse des Manipulators 22 angeordnet ist, so dass ihr Mittelpunkt in der Projektion auf die Bildebene des Flächendetektors 14 eine Trajektorie beschreibt, die beispielsweise eine Ellipse ist, während das Strahlungsquelle/Flächendetektor-Paar einerseits oder die Kugel andererseits um die Rotationsachse gedreht werden. Insbesondere bildet die Trajektorie der Kugelmittelpunktes in der Projektion eine geschlossene Bahn, die je nach Verkippung in einer Hälfte schneller durchlaufen wird als in der anderen Hälfte derselben und bei keiner Verkippung beispielsweise mit konstanter Geschwindigkeit bezogen auf den Rotationswinkel bzw. mit einem konstanten Abstand zwischen den bei äquidistanten Rotationswinkeln des Manipulators erzeugten Aufnahmen durchlaufen wird. In anderen Worten ausgedrückt wird beispielsweise der Rotationswinkel zwischen den Aufnahmen des Kalibrierobjekts mit gleicher Winkeldifferenz geändert und die entstehenden Segmentlängen zwischen den entsprechenden Mittelpunktspositionen auf der erwähnten Ellipse werden ausgewertet, um die Kippwinkel zu berechnen. Die Verwendung einer Kugel als Kalibrierobjekt stellt aber auch nur ein Beispiel für ein Kalibrierobjekt dar, für das eine ausgezeichnete Position - hier der Mittelpunkt - in der Projektionsebene im Rahmen des Schrittes 32 relativ einfach, und vor allem subpixelgenau, d.h. z.B. in einer Auflösung, die beispielsweise noch höher als die Auflösung der Urpixel 48 ist, bestimmt werden kann. Andere Kalibrierobjekte können ebenfalls einfach bestimmbare ausgezeichnete Positionen, also insbesondere auch mehr als eine, besitzen, wie z.B. Vielflächner, wie z.B. Pyramiden, Würfel oder dergleichen. Auf der anderen Seite kann beispielsweise eine Verdrehung des Flächendetektors 14 gegenüber seiner Solllage um die Flächennormalen der Projektionsebene des Flächendetektors 14, und zwar dem Sollnormalen Vektor, gegenüber beispielsweise einer Translationsverfahrachse des Manipulators 22 dadurch bestimmt werden, dass ein Kalibrierobjekt entlang dieser Translationsverfahrachse an zwei verschiedenen Positionen oder mehreren Positionen entlang dieser Translationsverfahrachse positioniert und aufgenommen wird. Auf diese Weise kann einfach der Winkel zwischen der Linie bestimmt werden, die die ausgezeichneten Punkte des Kalibrierobjektes in der Projektionsebene gegenüber beispielsweise der Zeilen- und/oder Spaltenrichtung von beispielsweise dem Pixelraster 52 einnehmen, um auf diese Weise eine Rückdrehung zu berechnen. Auf ähnliche Weise kann durch die zuvor beschriebene Verkippungsbestimmung durch Auswertung der vorbeschriebenen Positionsänderung beim Durchlaufen der geschlossenen Trajektorie in der Projektionsebene durch das Kalibrierobjekt der Winkel der Kippachse gegenüber beispielsweise der Spalten- oder Zeilenrichtung des Pixelrasters 52 und der Kippwinkel um diese in der Projektionseben verlaufende) Kippachse bestimmt werden, d.h. der Winkel der Verkippung um die Kippachse, die wiederum senkrecht zum Sollnormalen Vektor liegt. Wird wie in den folgenden Beispielen eine Kugel als Kalibrierobjekt verwendet, so ist es vorzuziehen, wenn die Kugel bezogen auf das Urpixelraster 48 oder das Pixelraster 52 beispielsweise eine ungefähre Größe einnimmt, so dass der Radius der Projektion dieser Kugel in die Projektionsebene des Flächendetektors 14 beispielsweise mehr als drei Pixel einnimmt, so dass durch Interpolation die Lage des Mittelpunktes Subpixel-genau bestimmt werden kann. Der Durchmesser kann beispielsweise 20 Pixel betragen.

Im Folgenden soll noch auf weitere mögliche Details eingegangen werden, die auch nachfolgend Bezug nehmend auf die Figuren 5-12 noch angesprochen werden.

Beispielsweise ist es möglich, dass es dem Benutzer des Durchstrahlungssystems 10 ermöglicht wird, die Auflösung der Durchstrahlungsbilder am Ausgang des Vorverarbeiters 24 variabel einzustellen. Die Korrekturdaten 28 können für jede dieser Einstellungsmöglichkeiten gesondert vorbestimmt sein oder für alle dieser Einstellungsmöglichkeiten gleich sein. Beispielsweise beschränkt sich die Einstellbarkeit der Auflösung der Durchstrahlungsbilder am Ausgang des Vorverarbeiters 24 auf eine reine Einstellbarkeit des Binning-Ausmaßes von der Auflösung, in der die geometrische Korrektur durchgeführt worden ist, zu der Auflösung der schließlichen Durchstrahlungsbilder. Beispielsweise befinden sich nach der Kalibrierung die Korrekturdaten 28 in einem Zustand, in dem sie für jedes Pixel 54 - in der geometrisch korrigierten Lage (innerhalb der Großpixel 58) - modifizierte Gewichtungsfaktoren besitzen, die angeben, wie sie aus den Urpixeln 48 zu kombinieren sind, und die Binningeinstellungsmöglichkeiten beschränken sich auf eine Einstellung der Größe der nxn-Blöcke, zu denen die so verschobenen Pendants der Pixel 54 zu den Grobpixeln 58 zusammengefasst werden. In Figur 4 wäre dann eine 3x3-Einstellung veranschaulicht, wobei aber auch lediglich ein 2x2-Binning möglich wäre. Dieses nachher einstellbare Binning wäre somit ortsinvariant über die Bildebene 36 hinweg.

Schließlich soll noch darauf hingewiesen werden, dass im Vorhergehenden häufig angenommen worden ist, dass die Durchstrahlungsbilder am Ausgang des Vorverarbeiters 24 in einem regelmäßigen Pixelraster vorliegen, das Spalten und Zeilen, beispielsweise in einer kartesischen Anordnung, aufweist. Das ist aber nicht unbedingt notwendig. Im Folgenden wird darauf hingewiesen werden, dass es aus unterschiedlichen Gründen vorteilhaft oder von dem Rekonstruierer 26 gefordert sein kann, dass die Pixelanordnungen anderen Regelmäßigkeiten gehorchen, wie z.B. äquiangular in einer Spalten- oder Zeilenrichtung angeordnet zu sein, oder in einer radialen Richtung mit somit einer polaren Anordnung der Pixel. Dies ist aber kein grundsätzliches Problem. Die Korrekturdaten 28 geben in diesem Fall immer für ein Pixel aus einer solchen allgemeineren regelmäßigen Pixelanordnung an, wie beispielsweise Urpixel 48, die mit dem jeweiligen Zielpixel in dem Durchstrahlungsbild zu kombinieren sind, d.h. mit welchen Gewichtungen, um das jeweilige Zielpixel der Durchstrahlungsbilder zu ergeben, wobei die geometrische Korrektur dabei in den Gewichtungen in einer Auflösung enthalten ist, die größer als die Endauflösung dieser Durchstrahlungsbilder ist.

Im Hinblick auf das Ausführungsbeispiel von Figur 3 und 4 mit der Mehrzahl von Kameras 38₁ und 38₂, die quasi als eine Mehrzahl von Flächendetektor-Arrays des Flächendetektors 14 fungieren, soll noch einmal darauf hingewiesen werden, dass es sein kann, dass die Korrekturdaten 28 in den Überlappungsregionen der Teilbilder 50 so eingestellt sind, dass sie für ein Pixel des regelmäßigen Pixelrasters der Durchstrahlungsbilder in einem Überlappungsbereich der Teilbilder 50 eine Kombination von Pixel definieren, die zu zwei unterschiedlichen benachbarten Teilbildern 50 gehören. Und zwar auch dann, wenn eigentlich dieses Grobpixel, wie z.B. das Pixel 58 in Figur 4, nur mit dem Überlappungsbereich bzw. nur in dem Überlappungsbereich mit einem benachbarten Teilbild 50 überlappt, aber ansonsten vollständig in einem Teilbild enthalten ist. Auf diese Weise werden die zur Verfügung stehenden Bildinformationen optimal ausgenutzt.

Die obigen Ausführungsbeispiele ermöglichen es also, unter Verwendung einer nicht-exakt ausgerichteten, aber beispielsweise einer bezüglich der Geometrie exakt charakterisierten, d.h. vorkalibrierten Kamera bzw. einem entsprechenden Flächendetektor 14, Bilder mit Pixelkoordinaten zu generieren, die bezüglich des Gesamtsystems und genauer dem Achsensystem 20 desselben, exakt ausgerichtet sind. Es sei darauf hingewiesen, dass sich die oben beschriebenen und auch noch weiter unten an konkreten Beispielen vorgestellten Geometriekorrekturen nicht ausschließlich auf die Kalibrierung von Röntgenkameras beschränkt, sondern auch für alle anderen Anwendungen eingesetzt werden kann, bei denen Pixeldaten in einer bestimmten Aufnahmegeometrie generiert werden müssen.

Wie es oben beschrieben worden ist, ist es durch eine geeignete geometrische Datentransformationsoperation in Schritt 32 möglich, aus den ursprünglichen Pixeldaten, d.h. den Pixelinformationen an den Pixeln 48 einer nicht-exakt ausgerichteten Kamera bzw. eines entsprechenden Flächendetektors 14, ein neues Bild zu gewinnen, das den Ansprüchen der jeweiligen Anwendung bezüglich der geometrischen Ausrichtung genügt, wie z.B. eben das Bild mit den Pixeln 56 in Figur 4. Dabei werden die neuen Pixelwerte des zu berechnenden transformierten Bildes aus den Pixelwerten der ursprünglichen Pixeldaten berechnet. Im einfachsten Fall werden die zu berechnenden Daten aus den Nachbarpixelwerten des ursprünglichen Bildes mit den Pixeln 48 mittels einer bilinearen Interpolation berechnet, und zwar nicht gleich für die Grobpixel 56, sondern für alle Pixel 54 in ihrer verschobenen geometrisch korrigierten Position. Im ungünstigsten Fall wird dadurch die Auflösung des Bildes halbiert. Nachher kann dann das Binning durchgeführt werden, wie es im Vorhergehenden beschrieben worden ist.

Die Vorkalibrierung kann beispielsweise wie in dem Patent DE 10 301 941 beschrieben durchgeführt werden, d.h. schon bei einer höheren Auflösung als der Zielauflösung, wobei erst danach die Auflösung auf das gewünschte Maß reduziert wird, d.h. durch das angesprochene Binning. Die entsprechenden Korrekturdaten werden dann modifiziert, wie es im Vorhergehenden Bezug nehmend auf Figur 2 in der Alternative mit dem Schritt 60 beschrieben worden ist. Insgesamt wird dadurch der Informationsverlust durch die geometrische Korrektur deutlich reduziert bzw. verhindert. Die geometrische Bildkorrektur und Zusammenfassung mehrerer Pixelwerte können auch, wie es im Vorhergehenden beschrieben worden ist, in einem einzigen Verarbeitungsschritt durchgeführt werden, d.h. das Binning und die geometrische Korrektur zur Lagekorrektur des Flächendetektors 14.

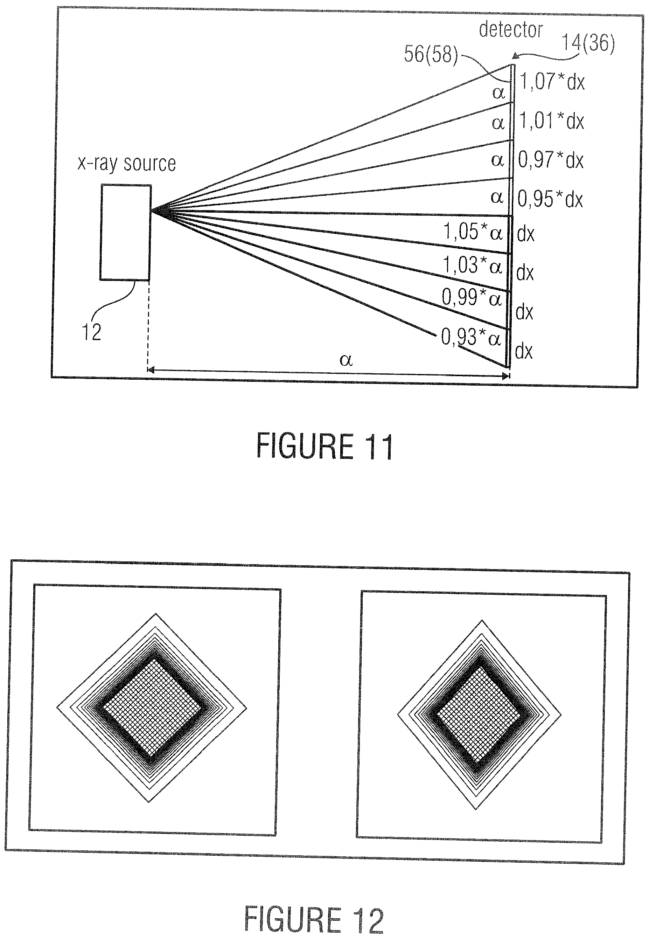

Im Rahmen der geometrischen Anpassung einer Aufnahme können auf die oben und später im Folgenden noch erörterte Weise bestimmte Pixelanordnungen erzeugt werden, die unterschiedlichsten Anforderungen genügen und von einer kartesischen Pixelanordnung abweichen können, was mit bisher eingesetzten Detektoren nicht ohne deutlichen Informationsverlust realisiert werden konnte:
1. Spalten und Zeilen stehen rechtwinklig aufeinander. Alle Pixel haben eine quadratische Form und dieselbe Größe. (Standard-Radioskopie-Fall)
2. Die Spalten und Zeilen des Bildes haben eine definierte kartesische Orientierung innerhalb einer Anordnung eines Gesamt-Systems (Röntgenquelle, mechanische Verfahrachsen). (Standard-CT-Fall)
3. Die Pixel sind nicht rechtwinklig, haben aber dieselbe Größe, die PixelKoordinaten stehen nicht senkrecht aufeinander. (z.B. Helix-CT-Anwendung)
4. Die Pixel besitzen eine rechteckige Form mit unterschiedlicher Höhe und Breite. Das Verhältnis von Höhe zu Breite kann jeden beliebigen Wert annehmen.
5. Die Pixel-Ausdehnung und -Koordinaten variieren abhängig von der Position auf dem Detektor.
6. Definierte lineare und nicht-lineare Bild-Verzerrung, d.h. die Orientierung der Zeilen und/oder Spalten sind abhängig von der Position auf dem Detektor. Dadurch kann beispielsweise eine virtuelle sphärische oder zylindrische Röntgenkamera mit Röntgenquellen-Punkt als Mittelpunkt erzeugt werden, wobei beispielsweise alle Pixel denselben Raumwinkel oder denselben Polarwinkel erfassen. (OPED-CT). Des Weiteren kann mit dem Verfahren eine reale nichtplanare Detektorfläche in eine virtuelle ideal-planare Detektorfläche umgewandelt werden. Im Fall der planaren Computer-Tomographie (PCT) kann abhängig vom eingestellten Einstrahlwinkel die Geometrie-Korrektur so betrieben werden, dass auch Durchstrahlungs-Projektionen mit einer realen nicht-planaren Detektorfläche zu korrekten Rekonstruktions-Ergebnissen führen. Ein weiteres Beispiel einer realisierbaren Bildtransformation ist, die Aufnahmen eines schräg stehenden Detektors so zu transformieren, dass diese den Aufnahmen eines senkrecht stehenden Detektors entsprechen. Auch der umgekehrte Fall, die Aufnahmen eines senkrecht stehenden Detektors zu transformieren, dass sie den Aufnahmen eines schräg stehenden Detektors entsprechen, ist möglich. Der Effekt einer Umtransformation zwischen einer kartesischen (äquidistant) und einer virtuell zylindrischen (äquiangular) Röntgenkamera ist in Fig. 11 angedeutet. Fig. 11 zeigt ein Beispiel eines Vergleichs zwischen einer äquidistanten (unten) und äquiangularen (oben) Pixelanordnung auf einer Bild- bzw. Projektionsebene 36 eines Detektors 14. Der untere Bereich des Detektors 14 ist in Pixel gleicher Größe *dx* unterteilt. Der obere Bereich des Detektors ist in Pixel unterteilt, die immer den gleiche Winkelbereich α abdecken. Im äquidistanten Bereich nimmt der abgedeckte Winkelbereich nach außen hin ab. Im äquiangularen Bereich des Detektors nimmt die Pixelgröße nach außen hin zu.
7. Transformation der Pixel-Geometrie, dass z.B. alle Bildpunkte mit demselben Abstand zum Zentrum des Detektors in einer Zeile abgebildet werden. Ein aufsteigender Zeilenindex entspricht dann dem wachsenden Abstand zum Zentrum des Detektors. Der Abstand kann als z.B. Abstand auf dem Detektor oder als Winkel zwischen dem Zentralstrahl und dem Strahl auf den entsprechenden Bildpunkt bestimmt werden. Der Spaltenindex entspricht dem Polarwinkel des Bildpunktes. Die hier beschriebene Abbildung kann als Abwicklung bezeichnet werden. (Röntgenbeugung / Diffraktometrie).

Wenn die Kalibrier- bzw. Korrekturdaten 28, d.h. die Kalibrierparameter, die die geometrische Transformation der Pixelkoordinaten beschreiben, für verschiedene Situationen ermittelt und abgespeichert werden, kann ein Durchstrahlungssystem ohne großen Aufwand umkonfiguriert werden, indem die entsprechenden Parameter in eine Software- oder Hardwarekomponente geladen werden.

Die obigen Ausführungsbeispiele also noch einmal anhand von konkreten Anwendungsfällen und in anderen Worten beschreibend kann also durch eine geeignete Vorgehensweise bestimmt werden, wie eine lageunkorrigierte Originalaufnahme durch einen Flächendetektor 14 transformiert werden muss, damit sie den Anforderungen des bildgebenden Gesamt-systems bzw. des Durchstrahlungssystems genügt. Diese Bestimmung wird im Schritt 32 aus Figur 2 durchgeführt. Beispielsweise kann durch Aufnahmen eines bekannten Objekts in unterschiedlichen bekannten Positionen oder Orientierungen bestimmt werden, in weleher Weise sich der Ist- und der Soll-Zustand der Aufnahme unterscheiden, d.h. die tatsächliche Lage des Flächendetektors 14 von ihrer Soll-Lage unterscheidet. Die Form des Referenzobjekts und die Art und Weise der Aufnahme hängt von der Art der Kalibrieraufgabe ab. Mit der aus den Aufnahmen gewonnenen Informationen kann die mathematische Form der durchzuführenden Korrekturoperation im Schritt 32 ermittelt werden. Die Transformation der Bilddaten, d.h. die Anwendung der Korrekturdaten 28, kann, wie im Vorhergehenden erwähnt, in einem speziellen Teil der Kamera bzw. des Flächendetektors 14 oder an einem Ort zwischen der Kamera 14 und Datenaufnahme-Rechner bzw. Konstruierer 26 oder direkt innerhalb des letzteren durchgeführt werden. Im Anschluss an diese geometrische Transformation kann dann gemäß einem Ausführungsbeispiel eine Zusammenfassung benachbarter Pixel zur gewünschten Pixelendgröße 56 (Binning) durchgeführt werden. Ändert sich die Geometrie des Gesamtsystems durch eine veränderte Position des Flächendetektors 14 oder einer anderen Komponente der Anlage 10, kann durch Laden von angepassten Kalibrierdaten 28 des Detektors 14 letzterer an die neue Situation angepasst werden.

Für die Röntgen-Computer-Tomografie (CT) muss der Flächendetektor 14 beispielsweise gegenüber dem mechanischen Achsensystem, wie er durch den Manipulator 22 festgelegt ist, exakt ausgerichtet werden und soll während der Datenaufnahme seine Position und auch die Orientierung nicht ändern, um Artefakte in der anschließenden Rekonstruktion des ausgenommenen Objekts in dem Rekonstruierer 26 zu vermeiden. In anderen Worten ausgedrückt kann es sich bei dem Durchstrahlungssystem 10 von Figur 1 beispielsweise um einen Computertomographen handeln, wobei Ziel der Installation des Systems ist, dass die Spalten des Flächendetektors 14 parallel zur Rotationsachse des Manipulators 22 ausgerichtet sind, der beispielsweise ein Objektmanipulator ist, d.h. ein Manipulator, der das Objekt relativ zur Strahlquelle 12 und Flächendetektor 14 bewegt. Wie es im Vorhergehenden beschrieben worden ist, wäre es natürlich umgekehrt auch möglich, das Objekt fest stehen zu lassen. Eine mögliche Verkippung des Detektors in die Strahlrichtung 20a und senkrecht dazu ist eigentlich bei der Installation so weit wie möglich zu vermeiden. Dies gilt beispielsweise für alle Konfigurationen von Strahlquelle bzw. Röntgenquelle 12, Manipulator 22 und Flächendetektor 14. Daraus resultieren mit steigender geforderter Detailauflösung des Systems 10 aufsteigende Anforderungen an die geometrische Genauigkeit aller mechanischen Komponenten, wie z.B. der Linear- und Rotationsachse des Systems 10. Ein CT-System gemäß Figur 1 erleichtert aber den Aufwand an der mechanischen Justierung, die ansonsten notwendig wäre, um die Orientierung der mechanischen Achsen und des Detektors einzustellen. Um das zu veranschaulichen, sei nachfolgend das Verfahren gemäß Figur 2 zur Ermittlung der Korrekturdaten 28 für das CT-Beispiel näher veranschaulicht. Es wird auf Figur 7 Bezug genommen. Ein zur Ermittlung der Korrekturdaten 28 geeignetes Objekt 70 wird relativ zur Rotationsachse bzw. auf der Rotationsachse 72, um die der Manipulator 22 das Paar aus Röntgenquelle 12 und Flächendetektor 14 oder das Objekt 70 rotiert, montiert. Ein geeignetes Objekt 70 ist beispielsweise eine Kugel aus stark absorbierendem Material in einem schwach absorbierenden Material oder ein anderes Objekt mit charakteristischen Strukturen, deren Positionen in Durchstrahlungsaufnahmen bestimmt werden können. In Figur 8 sind in der Konfiguration der CT-Anlage gemäß Figur 7 drei Sequenzen einer Kugel für verschiedene Aufnahmegeometrien gezeigt, d.h. für unterschiedliche Rotationswinkel um die Rotationsachse 72, alle eingezeichnet in ein Bild, und zwar links für eine unjustierte bzw. Verkippte Kamera 14b, in der Mitte für eine justierte Kamera 14a und rechts die Differenz beider Bilder. Das Objekt 70 wird also in unterschiedlichen Winkelpositionen um die Rotationsachse 72 aufgenommen (Schritt 30). Jeder Punkt des Referenzobjekts 70, d.h. der Mittelpunkt der Kugel, beschreibt dabei eine Kreisbahn. Die Projektion dieser Kreisbahn auf die Bildebene des Flächendetektors 14 ergibt eine Ellipse. Aus der Abweichung zwischen den gemessenen Bahnen der Referenzpunkte und denen für einen justierten Detektor in der Projektionsebene kann die geometrische Transformation in Schritt 32 ermittelt werden.

Ist der Flächendetektor 14 nämlich um die Strahlrichtung 20a verkippt, wird in dem Vorverarbeiter 24 das Rohbild, wie zum Beispiel das vorkalibrierte Bild mit dem Pixelraster 52 oder aber das Rohbild aus dem Pixel 48 direkt, um einen definierten Winkel gedreht, bevor die so erhaltenen Pixel, d. h. die kleineren Pixeln, die sich zu dem Pixel 56 in Figur 4 zusammensetzen, zur gewünschten Auflösung zusammengefasst werden (Binning). Ist der Detektor um die Spalten- und/oder die Zeilenrichtung der Pixel, wie zum Beispiel der Pixel 54, verkippt, entspricht die notwendige Bildkorrektur einer Entzerrung, wie es aus den Figuren 5 und 6 hervorgeht, die beispielsweise eben vor dem Binning durchgeführt wird. Durch eine derartige Bildkorrektur wird es möglich, auf besonders präzise und aufwendige mechanische Komponenten der Anlage teilweise zu verzichten und diese durch weniger präzise und aufwendige zu ersetzen, da die Anlage 10 an jede Situation durch eine Kalibrierung angepasst werden kann. Einzig die Rotationsachse 72 für das Objekt 70 muss weiterhin den Ansprüchen durch die gewünschte Auflösung genügen und entsprechend ein möglichst geringes Taumeln aufweisen.

Noch einmal in anderen Worten die Figuren 5 bis 8 beschreibend, zeigen diese Figuren also als Beispiel für ein Durchstrahlungssystem mittels einer Prinzipskizze einer Röntgenanlage. Dargestellt sind in Figuren 5 und 7 die Röntgenquelle 12 mit dem divergenten Strahlungskegel 74 der Strahlung 18. Der Detektor 14 ist in den Figuren 5 und 7 in zwei unterschiedlichen Orientierungen dargestellt, nämlich einmal 14a in der Solllage bzw. der idealen Orientierung, und das andere Mal 14b mit einem Lagefehler, nämlich hier exemplarisch einer seitlichen Verkippung gegenüber der Strahlachse 20a. Als Objekt 16 ist exemplarisch ein Würfel gewählt worden. Figur 6 zeigt die Simulation von durch Strahlungsaufnahmen eines Würfels in den zwei Stellungen 14a (rechts) und 14b (links). Die Simulationen entsprechen somit den beiden Detektororientierungen 14a und 14b in Fig. 5 bzw. 7. Links ist also eine seitliche Verkippung des Flächendetektors 14 simuliert worden, wohingegen rechts die Aufnahme bei justiertem (nicht verkipptem) Flächendetektor 14 dargestellt ist. Die Pfeile 76 verbinden einander korrespondierende Punkte in den Projektionen des Würfels 16.

Figur 7 veranschaulicht nun eine mögliche Anordnung zur Kalibrierung des dejustierten Flächendetektors 14 von Fig. 5. Dabei wird eine Sequenz von Aufnahmen einer Kugel als dem Kalibrierobjekt 70 erstellt. Für jede Einzelaufnahme wird die Kugel 70 um einen definierten Winkel um die Rotationsachse 72 weiterbewegt. Simulationen solcher Sequenzen sind in Figur 8 gezeigt. Insbesondere zeigt die Figur 8 simulierte Sequenzen von Röntgenaufnahmen der Kugel 70 in der Aufnahmegeometrie, wie sie in Figur 7 gezeigt ist. Im linken Teilbild ist simuliert worden, wie die Sequenz aussieht, wenn der Flächendetektor 14 in der Stellung 14b befindlich ist, d. h. seitlich verkippt ist. Das mittlere Bild zeigt das Ergebnis bei idealer Justierung des Flächendetektors 14, d. h. in der Stellung 14a. Rechts ist die Differenz zwischen der verkippten und der justierten Aufnahme dargestellt. Diese Differenz sorgte, falls sie nicht korrigiert würde, in einer anschließenden Verwendung der Durchstrahlungsbilder in dem Rekonstruierer 26 für einen Fehler, und in einer zwar korrigierten, aber eben nur in der Endauflösung korrigierten Form, verbliebe immer noch ein Teil des Fehlers in den Aufnahmen. Gemäß den obigen Ausführungsbeispielen wird dies dadurch verhindert, dass die Korrektur in einer höheren Auflösung stattfindet.

Nachfolgend sollen nun die Vorteile des erfindungsgemäßen Verfahrens für die planare Tomographie (PCT) gezeigt werden. Bei der planaren Tomographie werden bisher standardmäßig bei einem näherungsweisen planaren Objekt mit gleichbleibender Orientierung Röntgenquelle und Röntgenkamera relativ zum Objekt sequenziell in zueinander parallelen Ebenen positioniert, wobei die Achs-Ebenen parallel zur Hauptebene des Objekts ausgerichtet sind. Die Ebene der aktiven Fläche der Röntgenkamera wird ebenfalls parallel zu den übrigen Ebenen ausgerichtet. In anderen Worten ausgedrückt stellt beispielweise das System von Fig. 1 ein System für die planare Tomographie dar und es existiert ein Manipulator 22, der Translationsverfahrachsen aufweist, die zusammen eine Ebene aufspannen, bezogen auf welche das Strahlungsquellen/Flächendetektor-Paar einerseits und das Objekt, wie z.B. eine Leiterplatine, andererseits, gegenseitig translatorisch bewegt werden. Der Aufbau wird beispielsweise dazu verwendet, qualitativ schlechte Lötstellen zu erkennen. In der Solllage befindet sich die Projektionsebene des Flächendetektors 14 planparallel zu der Verfahrebene des Manipulators 22. Ist diese Ausrichtung erfolgt, kann aus der Verarbeitung von mehreren Projektionsaufnahmen aus unterschiedlichen Richtungen die Objektrekonstruktion erfolgen. Eine möglicherweise vorhandene Abweichung der aktiven Fläche der Röntgenkamera von der idealen Ebene führt im allgemeinen zu Fehlern, sogenannten Artefakten, bei der Objektrekonstruktion. Die Abweichung der Projektionsebene des Flächendetektors 14 von der idealen Ebene kann unterschiedliche Gründe haben, wie z.B. eine der im Vorhergehenden bereits erwähnten Gründe, wie z.B. die Verkippung gegenüber der Strahlachse, die Verdrehung gegenüber der Strahlachse bzw. den Manipulatorverfahrachsen oder dergleichen. Was hinzu kommen kann, ist eine Verzeichnung, die eventuell daher rührt, dass die Projektionsebene des Flächendetektors 14, wie z.B. der Schirm 36 des Szintillators von einer idealen planaren Form abweichend eine gewisse Krümmung besitzt. Letztere Abweichungsmöglichkeit gilt natürlich auch für alle anderen Ausführungsbeispiele. All solche Abweichungen von der Idealebene können entsprechend der beschriebenen Ausführungsbeispiele durch eine geeignete Kalibrierung korrigiert werden. Dies soll noch einmal konkret für den Anwendungsfall der planaren Tomographie beschrieben werden.

Als Objekt zur Ermittlung der Korrekturdaten 28 kann entweder eine ebene Platte aus stark absorbierendem Material mit definierten Löchern oder eine ebene Platte aus leicht absorbierendem Material mit Strukturen aus stark absorbierendem Material verwendet werden. Die Löcher bzw. Strukturen dienen als Referenzpunkte, die im Rahmen des Schrittes 32 erkannt werden können, bzw. deren Positionen in der Bildebene im Rahmen des Schrittes 32 bestimmt werden können, ebenso wie dies im Vorhergehenden im Fall von Figur 7 der Fall war. Die Platte muss anstelle des zu untersuchenden Objektes, wie zum Beispiel Leiterplatten, in der Anlage so positioniert werden, dass sie parallel zu den Ebenen der Detektor- und Röhrenbewegung liegt. Werden Detektor oder Röhre oder Objekt um eine definierte Strecke geradlinig verfahren, so beschreiben die gefundenen Positionen der Referenzpunkte in den Projektionen des Objekts auf dem Detektor mit einer regelmäßigen Anordnung quadratischer Pixel im Idealfall gerade Linienbahnen. Entspricht die Richtung der Bewegung der Soll-Ausrichtung der Detektorzeilen oder -Spalten können aus der Abweichung der Positionsänderung der projizierten Referenzpunkte zu einer Pixelzeile oder - Spalte die notwendigen Parameter zur Korrektur einer Dejustierung bestimmt werden. Werden Detektor oder Röhre oder Objekt wieder um die gleichen Strecken verfahren, wird die Projektion eines Referenzpunktes im Idealfall wieder um die gleiche Strecke verschoben wie im ersten Schritt. Beispielsweise wird zwischen den Aufnahmen des Kalibrierobjekts die Aufnahmekonstellation durch den Manipulator so geändert, dass sich bei idealer Lage und Ausrichtung des Detektors in der Projektionsebene eine Lage des ausgezeichneten Punkts des Kalibrierobjekts in der Projektionsebene ergäbe, die auf einem Quadrat liegen, und zwar beispielsweise parallel zu den Spalten und Zeilen des Detektors. Andere Vorgehensweisen wären natürlich ebenfalls möglich. Das geschieht in Schritt 30. Ändert sich die Länge und/oder die Richtung der Verschiebung der Projektion, ist die Ebene des Szintillatorschirms 36 nicht parallel zur Ebene, die durch die Verfahrachsen aufgespannt wird und/oder ist der Szintillatorschirm 36 nicht ideal planar. Aus den Abweichungen der Referenzpunktpositionen auf dem Detektor für unterschiedliche Röhren- oder Detektor- oder Objektpositionen kann die geometrische Korrektur der Bilddaten in Schritt 32 ermittelt werden, so dass sie den Anforderungen für die planare Tomographie genügen. Insbesondere kann ein Kalibrierobjekt mit einer ausreichend dichten Anordnung von Referenzpunkten dazu dienen die Verkippung oder Wölbung des Szintillatorschirms 36 zu bestimmen ohne eine Komponente der Anlage zu verfahren. Sind z.B. die Referenzpunkte äquidistant angeordnet, sollen auch die Projektionen der Referenzpunkte idealerweise äquidistant angeordnet sein. Das Kamerabild kann durch Rotation und ggf. Entzerrung vor dem Pixel-Binning gegenüber dem Achssystem in dem Vorverarbeiter justiert werden. Weiterhin ist auf die beschriebene Weise die Korrektur der Bildverzerrung aufgrund einer gewölbten Szintillatorfläche bei Schrägeinstrahlung durch Einbeziehung von a-priori-Informationen über den Einstrahlwinkel möglich. Gegebenenfalls muss für jede Detektorposition bzw. Strahlrichtung eine Korrektur des gewölbten Szintillators ermittelt und angewendet werden.

Ein weiteres Ausführungsbeispiel für das Durchstrahlungssystem von Figur 1 ist die als Ausgestaltung in Form einer Spiral-CT-Anlage. Bei der spiralen CT wird das Objekt bzw. Detektor 14 und Röntgenröhre als Strahlungsquelle 12 synchron zur Rotation des Objekts 16 in Richtung der Rotationsachse verschoben. Zur Rekonstruktion müssen die einzelnen Projektionen in diagonaler Richtung gefiltert werden. Der Winkel der Filterrichtung hängt wiederum von dem Verhältnis zwischen Rotations- und Vorschubgeschwindigkeit ab. Um die Filterung durchführen zu können, wird jede Aufnahme so mathematisch transformiert, dass die Zeilen parallel zur Filterrichtung ausgerichtet sind. Gemäß obigen Ausführungsbeispielen wird nun vermieden, dass ein Informationsverlust auftritt, weil die Transformation eben schon mit den hochaufgelösten Rohdaten durchgeführt wird, so dass der Informationsverlust durch die notwendige Rotation minimiert wird.

Für einen bestimmten CT-Rekonstruktionsalgorithmus (OPED; Orthogonal Polynomial Expansions on the Disk) werden Projektions-Aufnahmen benötigt, deren Pixel äquiangular bezüglich der Röntgenquelle angeordnet sind. Ein kreisförmig gebogener Detektor mit dem Kreismittelpunkt im Brennfleck der Röntgenquelle, dessen Pixel alle dieselbe Größe besitzen, würde diese Bedingung genau für einen bestimmten Abstand (bzw. Radius) zwischen Detektor und Röntgenquelle erfüllen. Andere Abstände zwischen Röntgenquelle und Detektor wären damit nicht zu realisieren. Ein planarer Detektor, dessen Pixel zum Rand hin entsprechend der Bedingung konstanter Winkelabstände kontinuierlich breiter werden, kann diese Bedingung erfüllen, wie es Fig. 11 in der oberen Hälfte des Detektors 14 angedeutet ist. Da die Pixelgröße mithilfe des hier beschriebenen Verfahrens variabel einstellbar ist, kann ein äqui-angulares Pixel-Format für alle möglichen Detektor-Quell-Abstände generiert werden, also so wie in der oberen Hälfte in Fig. 11 dargestellt. Das exakte benötigte Pixel-Format kann aus der Kenntnis des Detektor-Quell-Abstands d berechnet oder anhand von Kalibrieraufnahmen bestimmt und an die Anforderungen einer bestimmten Prüfaufgabe angepasst werden.

Bei der Pulverdiffraktometrie entstehen auf einem flachen Detektor ringförmige Beugungsmuster. Die Aufnahme kann geometrisch so transformiert werden, dass z.B. entlang der Spalten des ausgegebenen Bildes der radiale Grauwertverlauf für feste Polarwinkel angezeigt werden. Entsprechend wird dann der Grauwertverlauf für einen festen Abstand zum Zentrum und variablen Polarwinkel in einer Zeile dargestellt. Aus dieser Darstellung können die Daten zur Strukturanalyse einfacher gewonnen werden.

Seit einigen Jahren sind Röntgenkameras im Einsatz, die die optische Abbildung eines Röntgen-empfmdlichen Szintillatorschirms auf einen optische empfindlichen Bildsensors mittels Linsenoptiken nutzen. I.A. sind solche linsenoptisch erzeugte Bilder verzeichnet. In diesem Fall ist eine geometrische Korrektur einer Aufnahme zwingend notwendig, um ein unverzerrtes Bild zu erhalten. Wird die notwendige Verzeichnungskorrektur mit einer der oben beschriebenen geometrischen Transformationen zur Geometrie-Kalibrierung in einen einzigen Transformationsschritt kombiniert, ergibt sich kein weiterer Informationsverlust.

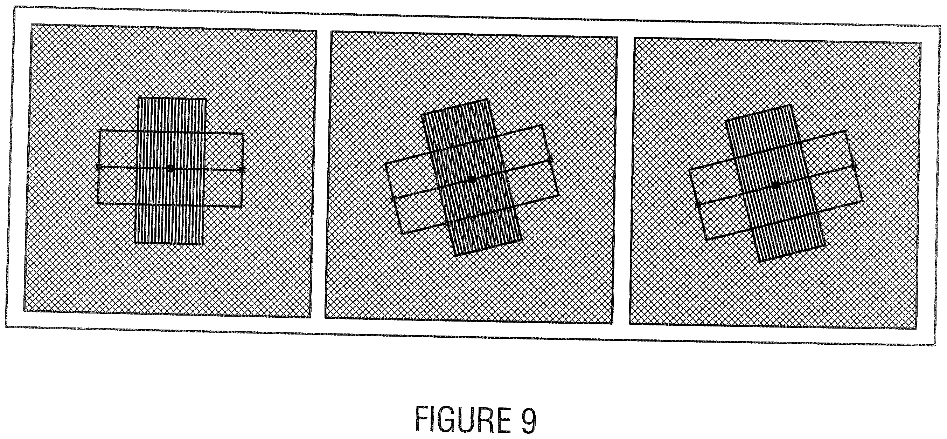

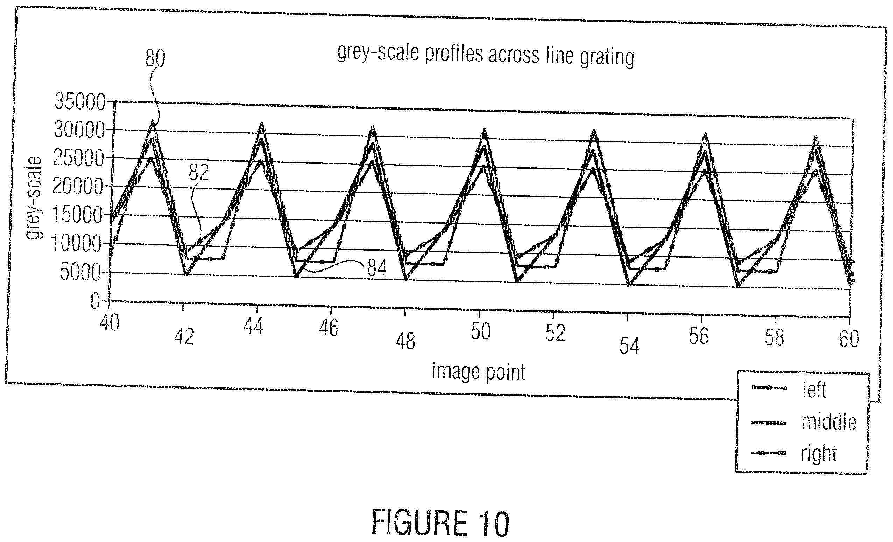

Schließlich zeigt Fig. 9 noch simulierte Aufnahmen eines Liniengitters, aufgenommen mit beispielsweise einer Konfiguration nach Fig. 5. Die Simulationen wurden zur Demonstration des Auflösungsverlustes durch geometrische Transformation bei grober Auflösung erstellt. Links ist die Aufnahme eines Liniengitters dargestellt, dessen Spaltbreite und - Abstände 1,5 Bildpunkte betragen. Das mittlere Bild zeigt die linke Aufnahme nach einer Rotation des Bildes um einen definierten Winkel. Für das rechte Bild wurde die Aufnahme bei der 4-fachen Auflösung simuliert, dann um denselben Winkel rotiert. Danach wurde die Auflösung auf ein Viertel reduziert. Entlang der roten Linien wurden Grauwertprofile erstellt die in Fig. 10 dargestellt sind. Das Profil 80 stellt den Helligkeitsverlauf über das linke und das Profil 82 über das mittlere Liniengitter von Fig. 9 dar. Es ist deutlich zu erkennen, dass durch die Rotation der Aufnahme Kontrast verloren wird. Das Profil 84 stellt den Helligkeitsverlauf über das rechte Gitter dar. Wird die Rotation der Aufnahme bei einer höheren Auflösung durchgeführt, verändert sich die Form des Profils, aber der Kontrast bleibt im Wesentlichen erhalten. Fig. 9 und 10 zeigen also, dass die obige Durchführung der geometrischen Korrektur bei einer Auflösung, die höher also die Endauflösung der Durchstrahlungsbilder ist, wie z.B. direkt an den unbehandelten Rohbildern des Flächendetektors 14 Vorteile in Bezug auf die Qualität der erhaltenen Durchstrahlungsbilder bzw. Aufnahmen hat.

Fig. 12 zeigt schließlich noch Simulationen der Durchstrahlungsaufnahme eines um 45° um die Strahlrichtung gedrehten Würfels. Links ist die Aufnahme des Würfels mit einem Detektor 14 dargestellt, dessen Pixel quadratisch sind. Rechts ist eine Aufnahme desselben Würfels dargestellt, bei der die Pixelbreite so variiert, dass die Pixel bezogen auf den Brennfleck der Röntgenquelle immer die gleiche Winkelbreite besitzen (vgl. Fig. 11). Hier erscheint die Aufnahme nach außen hin immer stärker gestaucht, da die reale Pixelgröße zunimmt (siehe auch Fig. 11).

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Verfahren zum Kalibrieren eines Durchstrahlungssystems, das eine Strahlungsquelle (12), einen Flächendetektor (14) zum Erzeugen von Durchstrahlungsrohbildern eines Objekts (16), einen Manipulator (22), der ausgebildet ist, eine relative Lage zwischen der Strahlungsquelle (12), dem Objekt (16) und dem Flächendetektor (14) zu ändern, wobei das Achssystem (20) durch zumindest eine Bewegungsachse (20b) des Manipulators (22) und eine Strahlachse (20a) zwischen der Strahlungsquelle (12) und dem Flächendetektor (14) definiert ist, und einen Vorverarbeiter (24) zum Anwenden von Korrekturdaten (28) auf die Durchstrahlungsrohbilder des Objekts (16), um Durchstrahlungsbilder des Objekts (16) zu erhalten, umfasst, mit
Erzeugen (30) von Durchstrahlungsrohbildern eines Kalibrierobjekts (70) in unterschiedlichen, durch den Manipulator (22) zueinander geänderten relativen Lagen zwischen der Strahlungsquelle (12), dem Kalibrierobjekt (70) und dem Flächendetektor (14) auf Basis von Strahlung (18), die das Kalibrierobjekt (70) zwischen der Strahlungsquelle (12) und dem Flächendetektor (14) durchdringt, durch den Flächendetektor (14); und
Neueinstellen der Korrekturdaten (28) abhängig von den Durchstrahlungsrohbildern des Kalibrierobjekts (70), so dass die Anwendung der neu eingestellten Korrekturdaten (28) zu einer geometrischen Korrektur der Durchstrahlungsrohbilder führt, so dass eine Veränderung einer Projektion des Objekts in dem Durchstrahlungsrohbild aufgrund eines Lagefehlers des Flächendetektors (14) in dem Achssystem (20) relativ zu einer Soll-Lage verringert wird, wobei der Lagefehler des Flächendetektors (14) definiert ist als
eine Verkippung einer Bildebene (36) des Flächendetektors (14) aus einer senkrechten Ausrichtung zu einer Strahlachse (20a) zwischen der Strahlungsquelle (12) und dem Flächendetektor (14), oder
eine Verkippung einer Spalten- oder Zeilenrichtung des Flächendetektors (14) relativ zu einer Rotations- oder Translationsverfahrachse des Manipulators (22) oder
eine Verkippung der Spalten- oder Zeilenrichtung des Flächendetektors (14) relativ zu einer Trajektorie, die der Flächendetektor (14) relativ zu dem Objekt (16) zwischen der Mehrzahl von Durchstrahlungsrohbildern abfährt,
**gekennzeichnet dadurch, dass**
der Vorverarbeiter (24) die Korrekturdaten (28) so auf die Durchstrahlungsrohbilder des Objekts (16) anwendet, dass die daraus erhaltenen Durchstrahlungsbilder des Objekts (16) eine geringere Auflösung als die Durchstrahlungsrohbilder des Objekts (16) aufweist, und dass
das Neueinstellen ein Neueinstellen der Korrekturdaten (28) abhängig von den Durchstrahlungsrohbildern des Kalibrierobjekts (70), so dass die Anwendung der neu eingestellten Korrekturdaten (28) zu einer geometrischen Korrektur der Durchstrahlungsrohbilder des Objekts (16) führt, so durchgeführt wird, dass die geometrische Korrektur in einer Auflösung stattfindet, die höher als die Auflösung der Durchstrahlungsbilder des Objekts (16) ist.

2. Durchstrahlungssystem mit
einer Strahlungsquelle (12);
einem Flächendetektor (14) zum Erzeugen von Durchstrahlungsrohbildern eines Objekts (16);
einem Manipulator (22), der ausgebildet ist, eine relative Lage zwischen der Strahlungsquelle (12), dem Objekt (16) und dem Flächendetektor (14) zu ändern, wobei das Achssystem (20) durch zumindest eine Bewegungsachse (20b) des Manipulators (22) und eine Strahlachse (20a) zwischen der Strahlungsquelle (12) und dem Flächendetektor (14) definiert ist;
einem Vorverarbeiter (24) zum Anwenden von Korrekturdaten (28) auf die Durchstrahlungsrohbilder des Objekts (16), um Durchstrahlungsbilder des Objekts (16) zu erhalten;
einer Vorrichtung zum Kalibrieren des Durchstrahlungssystems mit
einer Einrichtung zum Erzeugen (30) von Durchstrahlungsrohbildern eines Kalibrierobjekts (70) in unterschiedlichen, durch den Manipulator (22) zueinander geänderten relativen Lagen zwischen der Strahlungsquelle (12), dem Kalibrierobjekt (70) und dem Flächendetektor (14) auf Basis von Strahlung (18), die das Kalibrierobjekt (70) zwischen der Strahlungsquelle (12) und dem Flächendetektor (14) durchdringt, durch den Flächendetektor (14); und
einer Einrichtung zum Neueinstellen der Korrekturdaten (28) abhängig von den Durchstrahlungsrohbildern des Kalibrierobjekts (70), so dass die Anwendung der neu eingestellten Korrekturdaten (28) zu einer geometrischen Korrektur der Durchstrahlungsrohbilder führt, so dass eine Veränderung einer Projektion des Objekts (16) in dem Durchstrahlungsrohbild aufgrund eines Lagefehlers des Flächendetektors (14) in dem Achssystem (20) relativ zu einer Soll-Lage verringert wird, wobei der Lagefehler des Flächendetektors (14) definiert ist als
eine Verkippung einer Bildebene (36) des Flächendetektors (14) aus einer senkrechten Ausrichtung zu einer Strahlachse (20a) zwischen der Strahlungsquelle (12) und dem Flächendetektor (14), oder
eine Verkippung einer Spalten- oder Zeilenrichtung des Flächendetektors (14) relativ zu einer Rotations- oder Translationsverfahrachse des Manipulators (22) oder
eine Verkippung der Spalten- oder Zeilenrichtung des Flächendetektors (14) relativ zu einer Trajektorie, die der Flächendetektor (14) relativ zu dem Objekt (16) zwischen der Mehrzahl von Durchstrahlungsrohbildern abfährt,
**gekennzeichnet dadurch, dass** der Vorverarbeiter (24) so ausgebildet ist, dass derselbe die Korrekturdaten (28) so auf die Durchstrahlungsrohbilder des Objekts (16) anwendet, dass die daraus erhaltenen Durchstrahlungsbilder des Objekts (16) eine geringere Auflösung als die Durchstrahlungsrohbilder des Objekts (16) aufweist, und die Einrichtung zum Neueinstellen ausgebildet ist, um die Neueinstellung der Korrekturdaten (28) abhängig von den Durchstrahlungsrohbildern des Kalibrierobjekts (70), so dass die Anwendung der neu eingestellten Korrekturdaten (28) zu einer geometrischen Korrektur der Durchstrahlungsrohbilder des Objekts (16) führt, so durchzuführen, dass die geometrische Korrektur in einer Auflösung stattfindet, die höher als die Auflösung der Durchstrahlungsbilder des Objekts (16) ist.

3. Durchstrahlungssystem gemäß Anspruch 2, bei dem der Flächendetektor (14) eine Unregelmäßigkeit in der Verteilung von Pixelpositionen in einer Bildebene (36) des Flächendetektors (14) aufweist, und bei dem sich der Flächendetektors (14) in einem vorkalibrierten Zustand befindet, bei dem die Korrekturdaten (28) so voreingestellt sind, dass dieselben die Unregelmäßigkeit der Verteilung der Pixelpositionen in der Bildebene (36) korrigieren, so dass die Anwendung der so voreingestellten Korrekturdaten (28) zu einem regelmäßigen Pixelraster führt, die Einrichtung zum Erzeugen die Erzeugung in dem vorkalibrierten Zustand durchführt und die Einrichtung zum Neueinstellen bei dem Neueinstellen die Durchstrahlungsrohbilder des Kalibrierobjekts in einer Fassung verwendet, bei der die voreingestellte Korrekturdaten auf dieselben angewendet sind.

4. Durchstrahlungssystem gemäß Anspruch einem der Ansprüche 2 und 3, bei dem das Durchstrahlungssystem ferner einen Rekonstruierer (26) aufweist, der ausgebildet ist, um aus einer Mehrzahl von Durchstrahlungsbildern, die durch den Verarbeiter (24) aus Durchstrahlungsrohbildern erhalten worden sind, die von dem Flächendetektor (14) unter Veränderung der relativen Lage zwischen der Strahlungsquelle (12), dem Objekt (16) und dem Flächendetektor (14) zwischen den Durchstrahlungsrohbildern erzeugt worden sind, das Objekt (16) zu rekonstruieren, wobei der Rekonstruierer (26) ausgebildet ist, um bei der Rekonstruktion von einer Lagefehlerfreiheit des Flächendetektors (14) in dem Achssystem (20) auszugehen.

5. Durchstrahlungssystem gemäß Anspruch 4, bei dem die Einrichtung zum Neueinstellen ausgebildet ist, um das Neueinstellen so durchzuführen, dass die neu eingestellten Korrekturdaten (28) für jedes Pixel (56) der Durchstrahlungsbilder des Objekts (16) gemäß einer Vorschrift, die von Pixel zu Pixel des Durchstrahlungsbilds gemäß der geometrischen Korrektur variiert, festlegen, welche, und wie, benachbarte Pixel der Durchstrahlungsrohbilder des Objekts (16) zu dem jeweiligen Pixel zu kombinieren sind, und zwar unter Verwendung einer Gewichtung der Pixel der Durchstrahlungsrohbilder des Objekts (16), die von der Auflösung abhängt, in der die geometrische Korrektur durchgeführt wird.

6. Durchstrahlungssystem gemäß Anspruch 46, bei dem der Vorverarbeiter (24) und der Rekonstruierer (26) so ausgebildet sind, dass die Auflösung der Durchstrahlungsbilder des Objekts (16) über die Einstellung eines Binningausmaßes von der Auflösung, in der die geometrische Korrektur durchgeführt wird, zu der Auflösung der Durchstrahlungsbilder des Objekts (16) einstellbar ist.

7. Durchstrahlungssystem gemäß einem der vorhergehenden Ansprüche, bei dem die Einrichtung zum Neueinstellen ausgebildet ist, um das Neueinstellen so durchzuführen, dass die neu eingestellten Korrekturdaten (28) für jedes Pixel (54) eines regelmäßigen Pixelrasters (52) mit einer Zwischenauflösung, die gleich oder kleiner als die Auflösung der Durchstrahlungsrohbilder des Objekts (16) und höher oder gleich der Auflösung der Durchstrahlungsbilder des Objekts (16) ist, eine Kombination von Pixeln der Durchstrahlungsrohbilder des Objekts (16) bestimmen, die Pixel der Durchstrahlungsrohbilder des Objekts (16) umfassen, die flächenmäßig weniger als 100% mit dem jeweiligen Pixel des regelmäßigen Pixelrasters (52) überlappen.

8. Durchstrahlungssystem gemäß Anspruch 7, bei dem die Zwischenauflösung höher als die Auflösung der Durchstrahlungsbilder des Objekts (16) ist.

9. Durchstrahlungssystem gemäß Anspruch 7 oder 8, bei dem der Flächendetektor (14) eine Mehrzahl von Flächendetektorarrays (38₁, 38₂) zur Erzeugung von Teilbildern (50), die unter gegenseitigem Überlapp zusammen die Durchstrahlungsrohbilder des Objekts (16) ergeben, aufweist, wobei die Einrichtung zum Neueinstellen ausgebildet ist, um das Neueinstellen so durchzuführen, dass die neu eingestellten Korrekturdaten (28) ein Pixel des regelmäßigen Pixelrasters (52) als eine Kombination von Pixeln (48) zweier benachbarter Teilbilder (50) festlegen.

10. Durchstrahlungssystem gemäß Anspruch 7, bei dem ein ortsinvariantes Binning von der Zwischenauflösung zu der Auflösung der Durchstrahlungsbilder des Objekts (16) führt.

11. Computerprogramm mit einem Programmcode zur Durchführung des Verfahrens nach Anspruch 1, wenn das Programm auf einem Computer abläuft.

## Claims

1. Method for calibrating a radiation penetration system comprising a radiation source (12), a flat-panel detector (14) for generating radiation penetration raw images of an object (16), a manipulator (22) which is implemented to change a relative position between the radiation source (12), the object (16) and the flat-panel detector (14), wherein the axes system (20) is defined by at least one movement axis (20b) of the manipulator (22) and one beam axis (20a) between the radiation source (12) and the flat-panel detector (14), and a preprocessor (24) for applying correction data (28) to the radiation penetration raw images of the object (16) to obtain radiation penetration images of the object, comprising
generating (30) radiation penetration raw images of a calibration object (70) in different relative positions changed with respect to each other by the manipulator (22) between the radiation source (12), the calibration object (70) and the flat-panel detector (14) on the basis of radiation (18) which penetrates the calibration object (70) between the radiation source (12) and the flat-panel detector (14), by the flat-panel detector (14); and
readjusting the correction data (28) depending on the radiation penetration raw images of the calibration object (70) so that the application of the readjusted correction data (28) leads to a geometrical correction of the radiation penetration raw images, so that a change of a projection of the object in the radiation penetration raw image due to a position error of the flat-panel detector (14) in the axes system (20) relative to a necessitated position is reduced, wherein the position error of the flat-panel detector (14) is defined as
a tilting of an image plane (36) of the flat-panel detector (14) from a perpendicular orientation to a beam axis (20a) between the radiation source (12) and the flat-panel detector (14), or
a tilting of a column or line direction of the flat-panel detector (14) relative to a rotation or translation positioning axis of the manipulator (22), or
a tilting of the column or line direction of the flat-panel detector (14) relative to a trajectory along which the flat-panel detector (14) moves relative to the object (16) between the plurality of radiation penetration raw images,
**characterized in that**
the preprocessor (24) applies the correction data (28) to the radiation penetration raw images of the object (16) so that radiation penetration images of the object (16) obtained therefrom comprise a lower resolution than the radiation penetration raw images of the object (16) and that
readjusting executes a readjustment of the correction data (28) depending on the radiation penetration raw images of the calibration object (70) such that applying the readjusted correction data (28) leads to a geometrical correction of the radiation penetration raw images of the object (16), the geometrical correction taking place in a resolution which is higher than the resolution of the radiation penetration images of the object (16).

2. Radiation penetration system comprising
a radiation source (12);
a flat-panel detector (14) for generating radiation penetration raw images of an object (16);
a manipulator (22) which is implemented to change a relative position between the radiation source (12), the object (16) and the flat-panel detector (14), wherein the axes system (20) is defined by at least one movement axis (20b) of the manipulator (22) and one beam axis (20a) between the radiation source (12) and the flat-panel detector (14); and
a preprocessor (24) for applying correction data (28) to the radiation penetration raw images of the object (16) to obtain radiation penetration images of the object (16);
a device for calibrating the radiation penetration system, comprising:
means for generating (30) radiation penetration raw images of a calibration object (70) in different relative positions changed with respect to each other by the manipulator (22) between the radiation source (12), the calibration object (70) and the flat-panel detector (14) on the basis of radiation (18) which penetrates the calibration object (70) between the radiation source (12) and the flat-panel detector (14), by the flat-panel detector (14); and
means for readjusting the correction data (28) depending on the radiation penetration raw images of the calibration object (70) so that the application of the readjusted correction data (28) leads to a geometrical correction of the radiation penetration raw images, so that a change of a projection of the object (16) in the radiation penetration raw image due to a position error of the flat-panel detector (14) in the axes system (20) relative to a necessitated position is reduced, wherein the position error of the flat-panel detector (14) is defined as
a tilting of an image plane (36) of the flat-panel detector (14) from a perpendicular orientation to a beam axis (20a) between the radiation source (12) and the flat-panel detector (14), or
a tilting of a column or line direction of the flat-panel detector (14) relative to a rotation or translation positioning axis of the manipulator (22), or
a tilting of the column or line direction of the flat-panel detector (14) relative to a trajectory along which the flat-panel detector (14) moves relative to the object (16) between the plurality of radiation penetration raw images,
**characterized in that**
the preprocessor (24) is implemented such that the same applies the correction data (28) to the radiation penetration raw images of the object (16) such that the radiation penetration images of the object (16) obtained therefrom comprise a lower resolution than the radiation penetration raw images of the object (16), and the means for readjusting are implemented to execute the readjustment of the correction data (28) depending on the radiation penetration raw images of the calibration object (70), so that the application of the readjusted correction data (28) leads to a geometrical correction of the radiation penetration raw images of the object (16), so that the geometrical correction takes place in a resolution which is higher than the resolution of the radiation penetration images of the object (16).

3. Radiation penetration system according to claim 2, wherein the flat-panel detector (14) comprises an irregularity in the distribution of pixel positions in an image plane (36) of the flat-panel detector (14) and wherein the flat-panel detector (14) is in a pre-calibrated state, wherein the correction data (28) is preset such that the same correct the irregularity of distribution of the pixel positions in the image plane (36), so that the application of the thus preset correction data (28) leads to a regular pixel grid, the means for generating execute the generation in the pre-calibrated state and the means for readjusting use the radiation penetration raw images of the calibration object in the readjustment in a version wherein the preset correction data are applied to the same.

4. Radiation penetration system according to any one of claims 2 and 3, wherein the radiation penetration system further comprises a reconstructor (26) which is implemented to reconstruct the object from a plurality of radiation penetration images which were obtained by the processor (24) from radiation penetration raw images which were generated by the flat-panel detector (14) by changing the relative position between the radiation source (12), the object (16) and the flat-panel detector (14) between the radiation penetration raw images, wherein the reconstructor (26) is implemented to assume a position correctness of the flat-panel detector (14) in the axes system (20) in the reconstruction.

5. Radiation penetration system according to claim 4 wherein the means for readjusting are implemented to execute the readjustment such that the readjusted correction data (28) determine, for each pixel (56) of the radiation penetration images of the object (16), according to a specification which varies from pixel to pixel of the radiation penetration image in accordance with the geometric correction, which and how neighboring pixels of the radiation penetration raw images of the object (16) are to be combined to the respective pixel, i.e. using a weighting of the pixels of the radiation penetration raw images of the object (16), which depends on the resolution in which the geometrical correction is executed.

6. Radiation penetration system according to claim 4, wherein the preprocessor (24) and the reconstructor (26) are implemented such that the resolution of the radiation penetration images of the object (16) is adjustable by adjusting a binning extent from the resolution in which the geometrical correction is executed to the resolution of the radiation penetration images of the object (16).

7. Radiation penetration system according to any one of the preceding claims, wherein the means for readjusting are implemented to execute the readjustment such that the readjusted correction data (28) determine, for each pixel (54) of a regular pixel grid (52) with an intermediate resolution which is equal to or smaller than the resolution of the radiation penetration raw images of the object (16) and higher than or equal to the resolution of the radiation penetration images of the object(16), a combination of pixels of the radiation penetration raw images of the object (16) which comprise pixels of the radiation penetration raw images of the object (16) which overlap the respective pixel of the regular pixel grid (52) by less than 100% in area.

8. Radiation penetration system according to claim 7, wherein the intermediate resolution is higher than the resolution of the radiation penetration images of the object (16).

9. Radiation penetration system according to claim 7 or 8, wherein the flat-panel detector (14) comprises a plurality of flat-panel detector arrays (38₁, 38₂) for generating partial images (50) which, by mutually overlapping, together result in the radiation penetration raw images of the object (16), wherein the means for readjusting are implemented to execute readjustment such that the readjusted correction data (28) determine one pixel of the regular pixel grid (52) as a combination of pixels (48) of two neighboring partial images (50).

10. Radiation penetration system according to claim 7, wherein a location-invariant binning of the intermediate resolution leads to the resolution of the radiation penetration images of the object (16).

11. Computer program having a program code for executing the method according to claim 1 when the program runs on a computer.

## Revendications

1. Procédé d'étalonnage d'un système de rayonnement qui comporte une source de rayonnement (12), un détecteur de zone (14) destiné à générer des images brutes de rayonnement d'un objet (16), un manipulateur (22) qui est conçu pour modifier une position relative entre la source de rayonnement (12), l'objet (16) et le détecteur de zone (14), dans lequel le système d'axes (20) est défini par au moins un axe de déplacement (20b) du manipulateur (22) et un axe de faisceau (20a) entre la source de rayonnement (12) et le détecteur de zone (14), et un préprocesseur (24) destiné à appliquer des données de correction (28) aux images brutes de rayonnement de l'objet (16), pour obtenir des images de rayonnement de l'objet (16), avec le fait de
générer (30), par le détecteur de zone (14), des images de rayonnement brutes d'un objet d'étalonnage (70) dans différentes positions relatives modifiées l'une par rapport à l'autre par le manipulateur (22) entre la source de rayonnement (12), l'objet d'étalonnage (70) et le détecteur de zone (14) sur base du rayonnement (18) qui traverse l'objet d'étalonnage (70) entre la source de rayonnement (12) et le détecteur de zone (14); et
réajuster les données de correction (28) en fonction des images de rayonnement brutes de l'objet d'étalonnage (70), de sorte que l'application des données de correction réajustées (28) conduise à une correction géométrique des images de rayonnement brutes, de sorte que soit réduite une modification d'une projection de l'objet dans l'image de rayonnement brute par suite d'une erreur de position du détecteur de zone (14) dans le système d'axes (20) par rapport à une position de consigne, où l'erreur de position du détecteur de zone (14) est définie comme
une inclinaison d'un plan d'image (36) du détecteur de zone (14) à partir d'une orientation perpendiculaire à un axe de faisceau (20a) entre la source de rayonnement (12) et le détecteur de zone (14), ou
une inclinaison d'une direction de colonne ou de rangée du détecteur de zone (14) par rapport à un axe de déplacement en rotation ou en translation du manipulateur (22), ou
une inclinaison de la direction de colonne ou de rangée du détecteur de zone (14) par rapport à une trajectoire que parcourt le détecteur de zone (14) par rapport à l'objet (16) entre la pluralité d'images de rayonnement brutes,
**caractérisé par le fait que**
le préprocesseur (24) applique les données de correction (28) aux images de rayonnement brutes de l'objet (16) de sorte que les images de rayonnement de l'objet (16) ainsi obtenues présentent une résolution inférieure à celle des images de rayonnement brutes de l'objet (16), et que
le réajustement des données de correction (28) est effectué en fonction des images de rayonnement brutes de l'objet d'étalonnage (70) de sorte que l'application des données de correction réajustées (28) conduise à une correction géométrique des images de rayonnement brutes de l'objet (16), de sorte que la correction géométrique ait lieu avec une résolution qui est supérieure à la résolution des images de rayonnement de l'objet (16).

2. Système de rayonnement, avec
une source de rayonnement (12);
un détecteur de zone (14) destiné à générer des images de rayonnement brutes d'un objet (16);
un manipulateur (22) qui est conçu pour modifier une position relative entre la source de rayonnement (12), l'objet (16) et le détecteur de zone (14), où le système d'axes (20) est défini par au moins un axe de déplacement (20b) du manipulateur (22) et un axe de faisceau (20a) entre la source de rayonnement (12) et le détecteur de zone (14);
un préprocesseur (24) destiné à appliquer des données de correction (28) aux images de rayonnement brutes de l'objet (16) pour obtenir des images de rayonnement de l'objet (16);
un dispositif destiné à étalonner le système de rayonnement, avec
un dispositif destiné à générer (30), par le détecteur de zone (14), des images de rayonnement brutes d'un objet d'étalonnage (70) dans différentes positions relatives modifiées l'une par rapport à l'autre par le manipulateur (22) entre la source de rayonnement (12), l'objet d'étalonnage (70) et le détecteur de zone (14) sur base du rayonnement (18) qui traverse l'objet d'étalonnage (70) entre la source de rayonnement (12) et le détecteur de zone (14); et
un dispositif destiné à réajuster les données de correction (28) en fonction des images de rayonnement brutes de l'objet d'étalonnage (70), de sorte que l'application des données de correction réajustées (28) conduise à une correction géométrique des images de rayonnement brutes, de sorte que soit réduite une modification d'une projection de l'objet (16) dans l'image de rayonnement brute par suite d'une erreur de position du détecteur de zone (14) dans le système d'axes (20) par rapport à une position de consigne, où l'erreur de position du détecteur de zone (14) est définie comme
une inclinaison d'un plan d'image (36) du détecteur de zone (14) à partir d'une orientation perpendiculaire à un axe de faisceau (20a) entre la source de rayonnement (12) et le détecteur de zone (14), ou
une inclinaison d'une direction de colonne ou de rangée du détecteur de zone (14) par rapport à un axe de déplacement en rotation ou en translation du manipulateur (22), ou
une inclinaison de la direction de colonne ou de rangée du détecteur de zone (14) par rapport à une trajectoire que parcourt le détecteur de zone (14) par rapport à l'objet (16) entre la pluralité d'images de rayonnement brutes,
**caractérisé par le fait que**
le préprocesseur (24) est conçu de sorte qu'il applique les données de correction (28) aux images de rayonnement brutes de l'objet (16) de sorte que les images de rayonnement de l'objet (16) ainsi obtenues présentent une résolution inférieure à celle des images de rayonnement brutes de l'objet (16), et le dispositif destiné à réajuster est conçu pour effectuer le réajustement des données de correction (28) en fonction des images de rayonnement brutes de l'objet d'étalonnage (70) de sorte que l'application des données de correction réajustées (28) conduise à une correction géométrique des images de rayonnement brutes de l'objet (16), de sorte que la correction géométrique ait lieu avec une résolution qui est supérieure à la résolution des images de rayonnement de l'objet (16).

3. Système de rayonnement selon la revendication 2, dans lequel le détecteur de zone (14) présente une irrégularité dans la distribution des positions de pixels dans un plan d'image (36) du détecteur de zone (14), et dans lequel le détecteur de zone (14) se trouve dans un état pré-étalonné dans lequel les données de correction (28) sont préréglées de sorte qu'elles corrigent l'irrégularité de la distribution des positions de pixels dans le plan d'image (36), de sorte que l'application des données de correction ainsi préréglées (28) conduise à une grille de pixels régulière, que le dispositif destiné à générer effectue la génération à l'état pré-étalonné et que le dispositif destiné à réajuster utilise, lors du réajustement, les images de rayonnement brutes de l'objet d'étalonnage dans une version dans laquelle les données de correction préréglées sont appliquées à ces dernières.

4. Système de rayonnement selon les revendications 2 et 3, dans lequel le système de rayonnement présente par ailleurs un reconstructeur (26) qui est conçu pour reconstruire l'objet (16) à partir d'une pluralité d'images de rayonnement qui sont obtenues par le processeur (24) à partir des images de rayonnement brutes qui ont été générées par le détecteur de zone (14) en modifiant la position relative entre la source de rayonnement (12), l'objet (16) et le détecteur de zone (14) entre les images de rayonnement brutes, dans lequel le reconstructeur (26) est conçu pour partir, lors de la reconstruction, d'une absence d'erreurs de position du détecteur de zone (14) dans le système d'axes (20).

5. Système de rayonnement selon la revendication 4, dans lequel le dispositif destiné à réajuster est conçu pour effectuer le réajustement de sorte que les données de correction réajustées (28) établissent, pour chaque pixel (56) des images de rayonnement de l'objet (16), selon une règle qui varie d'un pixel à l'autre de l'image de rayonnement selon la correction géométrique, les pixels voisins des images de rayonnement brutes de l'objet (16) qui doivent être combinés avec le pixel respectif, et comment, et ce à l'aide d'une pondération des pixels des images de rayonnement de l'objet (16) qui dépend de la résolution avec laquelle est effectuée la correction géométrique.

6. Système de rayonnement selon la revendication 4, dans lequel le préprocesseur (24) et le reconstructeur (26) sont conçus de sorte que la résolution des images de rayonnement de l'objet (16) puisse être réglée en réglant une mesure de binning de la résolution avec laquelle est effectuée la correction géométrique à la résolution des images de rayonnement de l'objet (16).

7. Système de rayonnement selon l'une des revendications précédentes, dans lequel le dispositif destiné à réajuster est conçu pour effectuer le réajustement de sorte que les données de correction réajustées (28) pour chaque pixel (54) d'une grille de pixels régulière (52) avec une résolution intermédiaire qui est égale ou inférieure à la résolution des images de rayonnement brutes de l'objet (16) et supérieure ou égale à la résolution des images de rayonnement de l'objet (16) déterminent une combinaison de pixels des images de rayonnement brutes de l'objet (16) qui comprennent des pixels des images de rayonnement brutes de l'objet (16) qui viennent en chevauchement de moins de 100% en zone avec le pixel respectif de la grille de pixels régulière (52).

8. Système de rayonnement selon la revendication 7, dans lequel la résolution intermédiaire est supérieure à la résolution des images de rayonnement de l'objet (16).

9. Système de rayonnement selon la revendication 7 ou 8, dans lequel le détecteur de zone (14) présente une pluralité de réseaux de détecteurs de zone (38₁, 38₂) destinés à générer des images partielles (50) qui forment, ensemble, en chevauchement réciproque, les images de rayonnement brutes de l'objet (16), dans lequel le dispositif destiné à réajuster est conçu pour effectuer le réajustement de sorte que les données de correction réajustées (28) définissent un pixel de la grille de pixels régulière (52) comme une combinaison de pixels (48) de deux images partielles adjacentes (50).

10. Système de rayonnement selon la revendication 7, dans lequel un binning localement invariable conduit de la résolution intermédiaire à la résolution des images de rayonnement de l'objet (16).

11. Programme d'ordinateur avec un code de programme pour réaliser le procédé selon la revendication 1 lorsque le programme est exécuté sur un ordinateur.
